# EUROPEAN PATENT APPLICATION

(11) **EP 4 524 123 A1**
(43) Date of publication of application: **19.03.2025**
(21) Application number: 23802821.1
(22) Date of filing: 06.05.2023
(51) Int. Cl.: C07C 39/23, A61K 31/05, A61P 35/00, A61P 17/00, A61P 29/00

(54) **STILBENE DERIVATIVE AS AHR AGONIST AND USE THEREOF**

(30) Priority: 07.05.2022 CN 202210489648
(71) Applicant: Nanjing Gritpharmaco., Ltd., Nanjing, Jiangsu 211112 (CN)
(72) Inventor: CHEN, Lei, Nanjing, Jiangsu 211112 (CN); TIAN, Chao, Nanjing, Jiangsu 211112 (CN); ZHANG, Jilei, Nanjing, Jiangsu 211112 (CN); ZHU, Lijun, Nanjing, Jiangsu 211112 (CN); LU, Yingyan, Nanjing, Jiangsu 211112 (CN); ZHOU, Jing, Nanjing, Jiangsu 211112 (CN); WU, Xiaotao, Nanjing, Jiangsu 211112 (CN); LI, Zhan, Nanjing, Jiangsu 211112 (CN)
(74) Representative: Gille Hrabal Partnerschaftsgesellschaft mbB Patentanwälte
(86) International application number: PCT/CN2023/092595
(87) International publication number: WO 2023/217049

(57) **Abstract**

The present invention relates to a stilbene derivative as an AhR activator and use thereof. The compound of the present invention, or a pharmaceutically acceptable salt, a tautomer or a stereoisomer thereof has an excellent AhR activation effect, and can be safely used for treating a disease or a related symptom mediated by activity abnormality of AhR and related pathway targets.

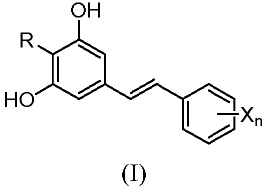

## Description

The present application claims priority to the prior Patent Application No. 202210489648.4 entitled "STILBENE DERIVATIVE AS AHR AGONIST AND USE THEREOF" and filed with China National Intellectual Property Administration on May 7, 2022, which is incorporated herein by reference in its entirety.

### TECHNICAL FIELD

The present disclosure relates to the field of pharmaceuticals, and particularly, to a stilbene derivative as an AhR agonist and use thereof.

### BACKGROUND

The aryl hydrocarbon receptor (AhR) is a member of the period-aryl hydrocarbon receptor nuclear translocator-single minded, Pre-Amt-Sim (b-HLH-PAS) subfamily, the basic helix-loop-helix (b-HLH) superfamily. It is distributed in various tissues and cells of the body, with the highest expression in spleen, stomach, ovary, and placenta, and is also highly expressed in immune cells. Particularly, the highest expression levels are observed in certain CD4⁺ T cell subsets, such as some hematopoietic stem cells, bone marrow-derived dendritic cells, and CD4⁺ Th17 cells, while the lowest levels are found in B cells and CD4⁺ Treg cells. The overactivation of Th17 cells and the increased secretion of IL-17 are associated with various chronic inflammatory diseases and autoimmune diseases in the body, such as psoriasis, multiple sclerosis, rheumatoid arthritis, ankylosing spondylitis, and asthma. Treg cells play an important role in suppressing autoimmune diseases, transplantation, and graft-versus-host disease. Studies have shown that the balance between Th17 and Treg plays an important role in host immunity and tolerance. For example, studies have shown that the pathogenesis of UC is closely related to the imbalance of Th17/Treg.

With the intensive research on AhR, the differentiation imbalance of Th17/Treg is found to be possibly related to *in vivo* AhR. The AhR is a transcription factor in cytoplasm that participates in regulating drug metabolism and cell growth and differentiation, and is closely related to the occurrence of immune response diseases and inflammatory diseases in the body. The activated AhR can regulate the differentiation of Th17 and Treg *in vivo.* A large number of experiments have demonstrated that the AhR is related to the occurrence of immune diseases in the body, such as asthma, smoking-related pulmonary inflammatory diseases, atopic dermatitis, chronic kidney disease, Sjogren's syndrome, inflammatory bowel disease, and the like.

ITE, TCDD, and FICZ are common AhR agonists, among which TCDD, the first discovered AhR agonist, can regulate the balance of T cell differentiation, but is limited in clinical use due to its environmental and *in vivo* toxicity. Benvitimod, as the first approved aryl hydrocarbon receptor agonist and a novel anti-inflammatory agent, can be used for treating various serious autoimmune diseases, such as psoriasis, eczema, purulent colitis, and various allergic diseases. As the first approved AhR agonist, benvitimod possesses unsatisfactory activity, and is prone to oxidization in light and thus difficult to preserve. Therefore, novel, improved compounds and compositions with better efficacy, higher bioavailability, and stronger stability are required in clinical applications.

### SUMMARY

In order to solve the technical problems described above, the present disclosure provides a compound of formula (I), or a pharmaceutically acceptable salt, a tautomer, or a stereoisomer thereof: 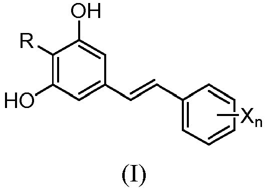
wherein X is selected from halogen, and n is an integer of 1-5; preferably, X is F or Cl, or X is a polysubstitution with F and Cl;
R is selected from substituted or unsubstituted 3- to 7-membered cycloalkyl or cycloalkenyl, and substituted or unsubstituted 3- to 7-membered heterocyclyl; the substituent is, for example: C₁₋₆ alkyl, C₁₋₆ alkoxy, halogen, and the like.

In some embodiments, X is one, two, or more of ortho, meta, and para substitutions.

In some embodiments, X is a monosubstitution with F, Cl, or Br.

In some embodiments, X is a disubstitution or trisubstitution with F.

In some embodiments, X is F, and n is 1.

In some embodiments, the substituted or unsubstituted 3- to 7-membered heterocyclyl is a N-containing heterocyclyl; the substitution is a substitution with at least one of C₁₋₆ alkyl, C₁₋₆ alkoxy, and halogen.

In some embodiments, the substituted or unsubstituted 3- to 7-membered cycloalkyl is cyclopentyl or cyclohexyl.

In some specific embodiments, the compound of formula (I) is selected from the following compounds:

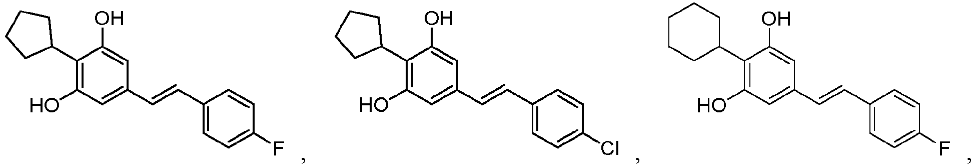

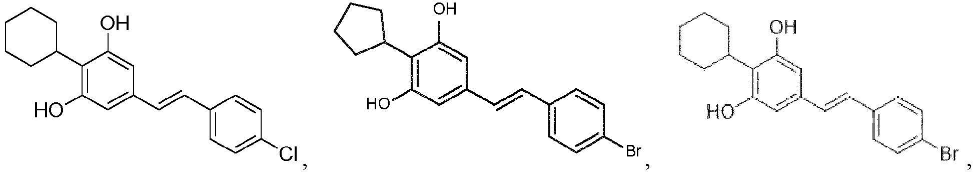

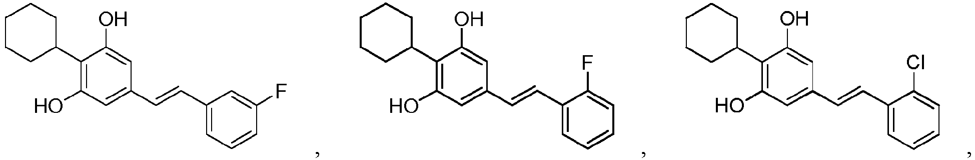

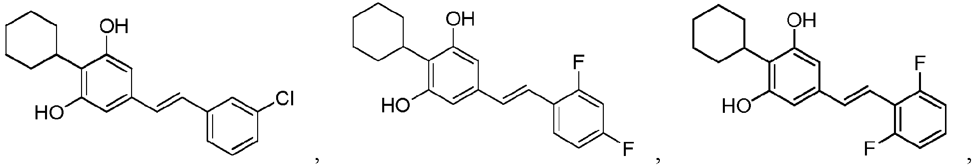

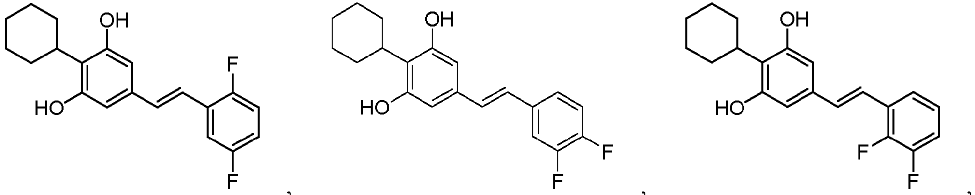

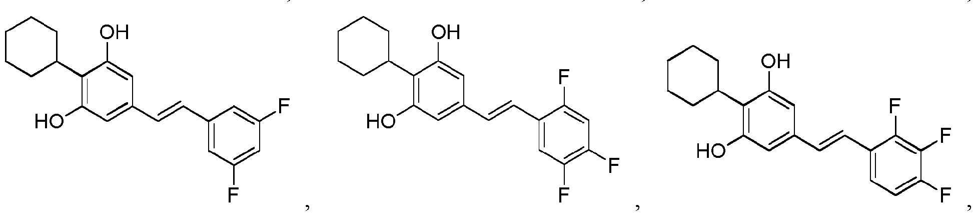

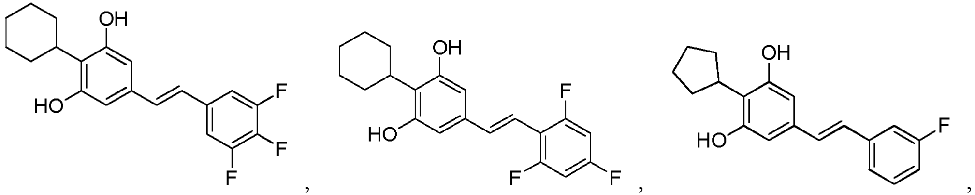

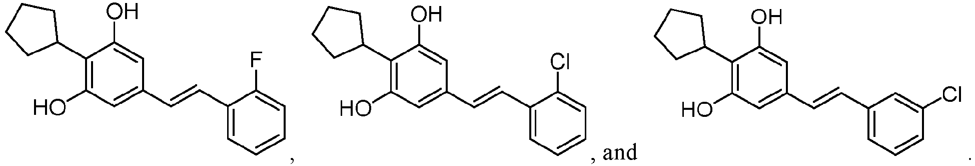

The present disclosure further provides a compound of formula (II), or a pharmaceutically acceptable salt, a tautomer, or a stereoisomer thereof:

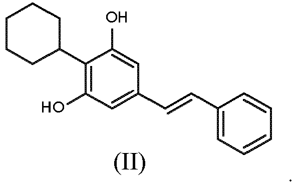

The present disclosure further provides use of the compound of formula (I) or formula (II), or the pharmaceutically acceptable salt, the tautomer, or the stereoisomer thereof for the manufacturing of a medicament for the treatment of a cancer, an autoimmune dysregulation, and other disorder with an immunological factor.

According to an embodiment of the present disclosure, the autoimmune dysregulation disorder is selected from one or more of psoriasis, eczema, atopic dermatitis, multiple sclerosis, systemic lupus erythematosus, inflammatory bowel disease, ulcerative colitis, rheumatoid arthritis, chronic kidney disease, ankylosing spondylitis, Sjogren's syndrome, polymyositis, vasculitis, polymyalgia rheumatica, immune thrombocytopenia, dry eye syndrome, type 1 diabetes, psoriasis, arthritis, and the like.

According to an embodiment of the present disclosure, the disorder with the immunological factor is selected from one or more of asthma, hypersensitivity, infection, osteoporosis, atherosclerosis, type 2 diabetes, graft-versus-host disease, and transplant rejection.

The present disclosure further provides use of the compound of formula (I) or formula (II), or the pharmaceutically acceptable salt, the tautomer, or the stereoisomer thereof for the manufacturing of a medicament for the prevention and/or treatment of a disease or condition mediated by the aryl hydrocarbon receptor (AhR).

The present disclosure further provides use of the compound of formula (I) or formula (II), or the pharmaceutically acceptable salt, the tautomer, or the stereoisomer thereof for the manufacturing of a medicament for the regulation of an immune- or inflammation-associated cytokine selected from IL-2, IL-3, IL-4, IL-5, IL-6, IL-10, IL-12, IL-13, IL-17, IL-22, IL-23, TNFα, TGF-β, IFN-γ, and IL-1β, and for the prevention and/or treatment of a disease caused by the abnormality of the cytokine described above.

The present disclosure further provides a pharmaceutical composition comprising the compound of formula (I) or formula (II), or the pharmaceutically acceptable salt, the tautomer, or the stereoisomer thereof, and a pharmaceutically acceptable auxiliary material, such as a carrier, an excipient, or the like.

The present disclosure further provides a method of treating and/or preventing a disorder or a disease mediated by the aryl hydrocarbon receptor (AhR), comprising: administering to a subject a therapeutically effective amount of a compound of formula (I) or formula (II), or a pharmaceutically acceptable salt, a tautomer, or a stereoisomer thereof.

The present disclosure further provides a method for preparing a compound of formula (I):
reacting compound IMe with compound SMc to give compound IMf, and reacting compound IMf in an acidic condition to give a compound represented by formula (I); 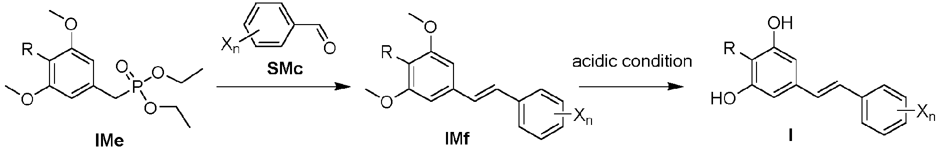
wherein X is selected from halogen, and n is an integer of 1-5;
R is selected from substituted or unsubstituted 3- to 7-membered cycloalkyl or cycloalkenyl, and substituted or unsubstituted 3- to 7-membered heterocyclyl.

Further, the preparation process of compound IMe is as follows:

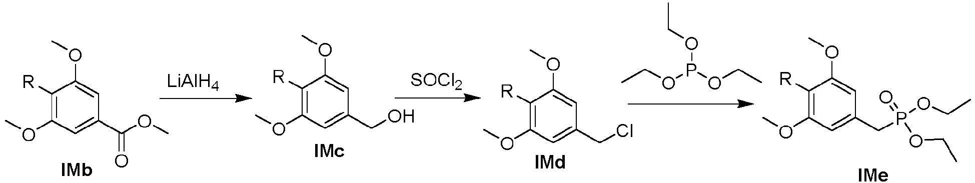

R is selected from substituted or unsubstituted 3- to 7-membered cycloalkyl or cycloalkenyl, and substituted or unsubstituted 3- to 7-membered heterocyclyl.

### Terminology

The "3- to 7-membered cycloalkyl or cycloalkenyl" used herein includes, but is not limited to, "3- to 7-membered monocyclic cycloalkyl or monocyclic cycloalkenyl", and specific examples include, but are not limited to: substituted or unsubstituted cyclopropyl, cyclobutyl, cyclopentyl, cyclopentenyl, cyclohexyl, cyclohexenyl, cycloheptyl, cycloheptenyl, and the like.

The "3- to 7-membered heterocyclyl" used herein refers to a saturated or partially saturated monocyclic group containing at least one heteroatom (e.g., 1, 2, 3, or 4 heteroatoms) and having 3-7 ring atoms, where the heteroatom is a nitrogen atom, an oxygen atom, and/or a sulfur atom.

The "stereoisomer" used herein refers to the compound of the present disclosure when it contains one or more asymmetric centers and thus can be present as a racemate and a racemic mixture, a single enantiomer, a diastereomeric mixture, and a single diastereoisomer. The compound of the present disclosure may have asymmetric centers that each independently produces two optical isomers. The scope of the present disclosure encompasses all possible optical isomers and mixtures thereof.

The "pharmaceutically acceptable carrier" refers to a carrier that can be used to prepare a pharmaceutical composition and is generally compatible with other components of the composition, harmless to the recipient, and neither biologically nor otherwise undesirable. The "pharmaceutically acceptable carrier" includes one and/or more carriers. Examples include carriers for topical, ocular, parenteral, intravenous, intraperitoneal, intramuscular, sublingual, nasal, and oral administration. The "pharmaceutically acceptable carrier" also includes reagents used in the preparation of aqueous dispersions and sterile powders for injection or dispersion.

As used herein, the "excipient" includes physiologically compatible additives that can be used in the preparation of a pharmaceutical composition. Examples of the pharmaceutically acceptable carrier and excipient can be found, for example, in Remington Pharmaceutical Science (16th edition).

### Beneficial Effects

Compared with the prior art, the present disclosure has the following advantages:
(1) The compound, the pharmaceutically acceptable salt, the tautomer, or the stereoisomer thereof disclosed herein has an excellent AhR agonistic effect and is safe in use for treating a disease mediated by AhR activity abnormality or a related disorder. The compound disclosed herein has been tested for the AhR agonistic activity at a concentration that has been confirmed with no significant effect on the viability of HepG2 cells. The results show that the compound disclosed herein exhibited an AhR agonistic activity 2-9 times those of the positive controls benvitimod and FICZ, suggesting a good effect in treating the disease mediated by AhR activity abnormality or the related disorder. In a DSS ulcerative colitis mouse model, the therapeutic effect of the compound disclosed herein was better than that of the control group at a dose of 10 mg/kg.
(2) The compound, the pharmaceutically acceptable salt, the tautomer, or the stereoisomer thereof disclosed herein has good biological stability and metabolic stability, good pharmacokinetic properties, and thus good clinical application prospects.
(3) The compound, the pharmaceutically acceptable salt, or the stereoisomer thereof disclosed herein shows relatively low toxicity, good tolerability, and good safety.
(4) The compound disclosed herein has high stability and good resistance to chemical degradation reactions such as hydrolysis, oxidation, and the like, with no color change or increase in impurity after long-term preservation at room temperature, no requirement for special in-dark preservation conditions, and significantly reduced photolysis in light as compared with benvitimod.

### BRIEF DESCRIPTION OF THE DRAWINGS

FIG. 1 illustrates the effect of the test compounds on the cell viability of PBMCs at a concentration of 1 µM;
FIG. 2 illustrates the effect of the test compounds on the cell viability of HepG2 cells at a concentration of 1 µM;
FIG. 3 illustrates the effect of the test compounds on colon length in the DSS ulcerative colitis mouse model (**p < 0.01 compared with the blank group; #p < 0.05 compared with the modeling group);
FIG. 4 illustrates the effect of the test compounds on disease activity index in the DSS ulcerative colitis mouse model (**p < 0.01 compared with the blank group; #p < 0.05 and ##p < 0.01 compared with the modeling group).
FIG. 5 illustrates the back of the mice in each group.
FIG. 6 illustrates the results of the 24-hour drug permeation rate test.
FIG. 7 illustrates the PASI scoring curves.

### DETAILED DESCRIPTION

The technical solutions of the present disclosure will be further described in detail with reference to the following specific examples. It will be appreciated that the following examples are merely exemplary illustrations and explanations of the present disclosure and should not be construed as limiting the claimed scope of the present disclosure. All techniques implemented on the basis of the content described above of the present disclosure are encompassed within the claimed scope of the present disclosure.

Unless otherwise stated, the starting materials and reagents used in the following examples are all commercially available products or can be prepared by using known methods.

### Example 1

The following method describes in detail the preparation of the compound disclosed herein. The compound disclosed herein and the compounds in the comparative examples can be prepared by those skilled in the art of organic synthesis using known or commercially available starting materials and reagents.

The following synthesis method illustrates the specific synthetic route for the compound of formula (I) disclosed herein: 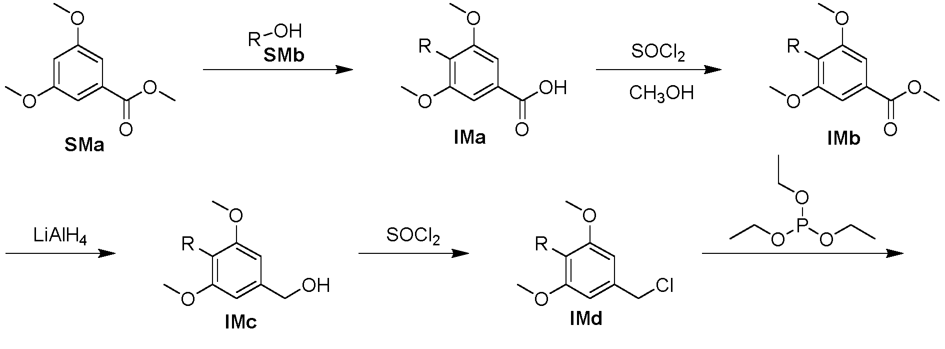 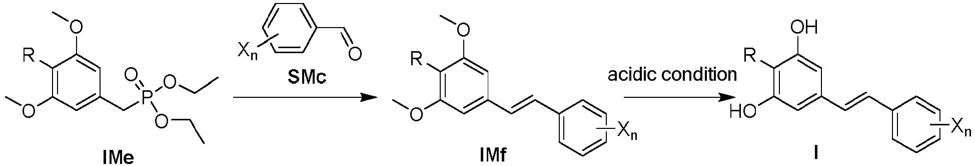
wherein X is selected from halogen, and n is an integer of 1-5; preferably, X is F or Cl, or X is a polysubstitution with F and Cl;
R is selected from substituted or unsubstituted 3- to 7-membered cycloalkyl or cycloalkenyl, and substituted or unsubstituted 3- to 7-membered heterocyclyl.

### Example 2

### Synthesis of diethyl 4-cyclopentyl-3,5-dimethoxybenzylphosphonate

### (1) Synthesis of 4-cyclopentyl-3,5-dimethoxybenzoic acid

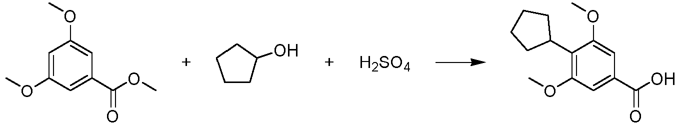

Methyl 3,5-dimethoxybenzoate (20.07 g, 102.29 mmol) was added to a single-neck flask before concentrated sulfuric acid (50 mL) was added. The mixture was stirred for dissolving. In an ice bath, cyclopentanol (21.95 g, 254.82 mmol) was added dropwise to the single-neck flask. After the addition of cyclopentanol, the ice bath was removed, and the reaction system was warmed to 70 °C and incubated for 4 h. The reaction was monitored by TLC and stopped after the depletion of the starting materials was confirmed. The reaction solution was cooled to room temperature and poured into a beaker. Saturated sodium bicarbonate solution was added to the beaker to adjust the reaction solution to pH 3-5. Three extractions were performed with ethyl acetate (200 mL) and water (200 mL). The ethyl acetate phases were combined, washed with saturated sodium chloride solution (100 mL), and dried over anhydrous sodium sulfate. After the drying was complete, ethyl acetate was removed by concentration at reduced pressure to give a crude brown solid product 4-cyclopentyl-3,5-dimethoxybenzoic acid (24.76 g). Yield: 96.7%.

### (2) Synthesis of methyl 4-cyclopentyl-3,5-dimethoxybenzoate

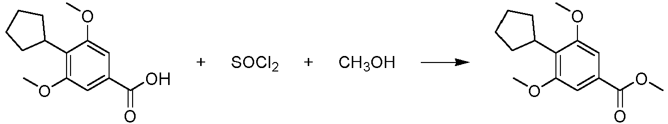

4-Cyclopentyl-3,5-dimethoxybenzoic acid (24.76 g, 98.92 mmol) was added to a single-neck flask before methanol (50 mL) was added. The mixture was stirred for dissolving. In an ice bath, thionyl chloride (17.65 g, 148.39 mmol) was added dropwise to the single-neck flask, with white smoke observed. After the addition of thionyl chloride, the ice bath was removed, and the reaction system was warmed to 60 °C and incubated for 1 h. The reaction was monitored by TLC and stopped after the depletion of the starting materials was confirmed. The reaction solution was cooled to room temperature and concentrated at reduced pressure to remove methanol and excess thionyl chloride. Three extractions were performed with ethyl acetate (150 mL) and water (150 mL). The ethyl acetate phases were combined, washed with saturated sodium chloride solution (100 mL), and dried over anhydrous sodium sulfate. After drying, ethyl acetate was removed by concentration at reduced pressure to give a crude black solid product methyl 4-cyclopentyl-3,5-dimethoxybenzoate (25.95 g). Yield: 99.2%.

Methyl 4-cyclopentyl-3,5-dimethoxybenzoate was purified by column chromatography (mobile phase: petroleum ether:ethyl acetate = 15:1). The mobile phase was concentrated to give a purified yellow solid product methyl 4-cyclopentyl-3,5-dimethoxybenzoate (18.54 g). Purification yield: 71.4%.

### (3) Synthesis of 4-cyclopentyl-3,5-dimethoxybenzyl alcohol

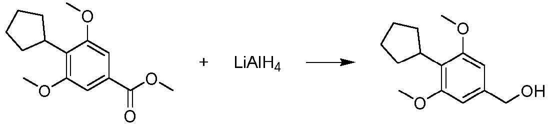

Methyl 4-cyclopentyl-3,5-dimethoxybenzoate (18.54 g, 70.14 mmol) was added to a single-neck flask before tetrahydrofuran (50 mL) was added. The mixture was stirred for dissolving. In an ice bath, lithium aluminum hydride (3.99 g, 105.21 mmol) was added in batches to the single-neck flask, with a large amount of bubbles observed. After the addition of lithium aluminum hydride, the ice bath was removed, and the reaction system was incubated at room temperature for 1.5 h. The reaction was monitored by TLC and stopped after the depletion of the starting materials was confirmed. In an ice bath, water (3.99 mL), 15% sodium hydroxide solution (3.99 mL), and water (11.97 mL) were sequentially added dropwise to the single-neck flask, and the reaction solution was stirred for 30 min, with a large amount of white solid precipitated. The white precipitate was removed by filtration through celite, and the filtrate was collected. The precipitate was washed with a small amount of tetrahydrofuran and filtered, and the washing was collected. The filtrate and the washing were combined and concentrated at reduced pressure to remove tetrahydrofuran. Three extractions were performed with ethyl acetate (50 mL) and water (50 mL). The ethyl acetate phases were combined, washed with saturated sodium chloride solution (30 mL), and dried over anhydrous sodium sulfate. After drying, ethyl acetate was removed by concentration at reduced pressure to give a crude brown solid product 4-cyclopentyl-3,5-dimethoxybenzyl alcohol (15.37 g). Yield: 92.7%.

### (4) Synthesis of 4-cyclopentyl-3,5-dimethoxybenzyl chloride

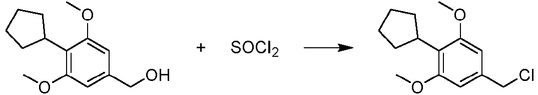

4-Cyclopentyl-3,5-dimethoxybenzyl alcohol (15.37 g, 65.04 mmol) was added to a single-neck flask before dichloromethane (30 mL) was added. The mixture was stirred for dissolving. In an ice bath, thionyl chloride (11.61 g, 97.56 mmol) was added dropwise to the single-neck flask, with white smoke observed. After the addition of thionyl chloride, the ice bath was removed, and the reaction system was incubated at room temperature for 2 h. The reaction was monitored by TLC and stopped after the depletion of the starting materials was confirmed. The reaction solution was concentrated at reduced pressure to remove dichloromethane and excess thionyl chloride. Three extractions were performed with ethyl acetate (50 mL) and water (50 mL). The ethyl acetate phases were combined, washed with saturated sodium chloride solution (30 mL), and dried over anhydrous sodium sulfate. After drying, ethyl acetate was removed by concentration at reduced pressure to give a crude black oily product 4-cyclopentyl-3,5-dimethoxybenzyl chloride (15.14 g). Yield: 91.4%.

4-Cyclopentyl-3,5-dimethoxybenzyl chloride was purified by column chromatography (mobile phase: petroleum ether:ethyl acetate = 100:1). The mobile phase was concentrated to give a purified yellow solid product 4-cyclopentyl-3,5-dimethoxybenzyl chloride (12.60 g). Purification yield: 83.2%.

### (5) Synthesis of diethyl 4-cyclopentyl-3,5-dimethoxybenzylphosphonate

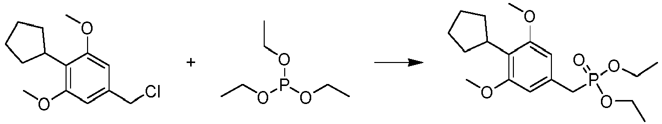

4-Cyclopentyl-3,5-dimethoxybenzyl chloride (12.60 g, 49.46 mmol) was added to a single-neck flask before triethyl phosphite (49.31 g, 296.76 mmol) was added. The mixture was stirred for dissolving. The reaction system was purged with nitrogen thrice, warmed to 160 °C, and incubated for 5 h. The reaction was monitored and stopped after the depletion of the starting materials was confirmed. The solvent was removed to give diethyl 4-cyclopentyl-3,5-dimethoxybenzylphosphonate (16.75 g). Yield: 95%.

### Example 3

### Synthesis of (E)-2-cyclopentyl-5-styryl-1,3-benzenediol

### (1) Synthesis of (E)-2-cyclopentyl-1,3-dimethoxy-5-styrylbenzene

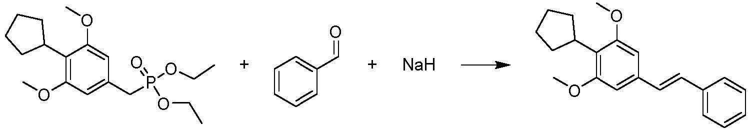

Diethyl 4-cyclopentyl-3,5-dimethoxybenzylphosphonate (1.01 g, 2.83 mmol) was added to a three-necked flask before tetrahydrofuran (15 mL) was added. The mixture was stirred in an ice bath for dissolving. Sodium hydride (452.5 mg, 11.31 mmol) was added in batches into the three-necked flask in a nitrogen atmosphere, and after the addition, the reaction system was incubated for 1 h. A solution of benzaldehyde (4.24 mmol) in tetrahydrofuran (450.2 mg of benzaldehyde in 10 mL of tetrahydrofuran) was slowly and dropwise added to the three-necked flask, and the ice bath was removed after the addition. The reaction system was slowly warmed to 70 °C and incubated for 4 h. The reaction was monitored by TLC and cooled to room temperature after the depletion of the starting materials. In an ice bath, water was added to the three-necked flask to quench the reaction until no gas was produced, and tetrahydrofuran was removed by concentration at reduced pressure. Three extractions were performed with ethyl acetate (30 mL) and water (30 mL). The ethyl acetate phases were combined, washed with saturated sodium chloride solution (20 mL), and dried over anhydrous sodium sulfate. After drying, ethyl acetate was removed by concentration at reduced pressure to give a crude yellow oily product (*E*)-2-cyclopentyl-1,3-dimethoxy-5-styrylbenzene (804.6 mg).

(*E*)-2-Cyclopentyl-1,3-dimethoxy-5-styrylbenzene was purified by column chromatography (mobile phase: petroleum ether:ethyl acetate = 50:1). The mobile phase was concentrated to give a purified yellow solid product (*E*)-2-cyclopentyl-1,3-dimethoxy-5-styrylbenzene (403.2 mg). Yield: 46.2%.

### (2) Synthesis of (E)-2-cyclopentyl-5-styryl-1,3-benzenediol

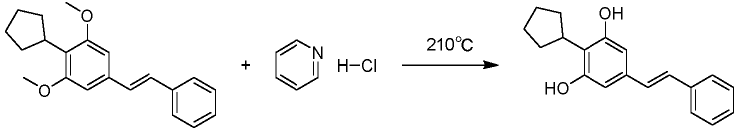

(*E*)-2-cyclopentyl-1,3-dimethoxy-5-styrylbenzene (403.2 mg, 1.31 mmol) and pyridine hydrochloride (3.02 g, 26.15 mmol) were added to a single-neck flask. The reaction system was purged with nitrogen thrice, slowly warmed to 210 °C, and incubated for 4 h after pyridine hydrochloride was melted. The reaction was monitored by TLC and stopped after the depletion of the starting materials was confirmed. The reaction solution was cooled to room temperature and extracted thrice with ethyl acetate (30 mL) and water (30 mL). The ethyl acetate phases were combined, washed with saturated sodium chloride solution (20 mL), and dried over anhydrous sodium sulfate. After drying, ethyl acetate was removed by concentration at reduced pressure to give a crude product (*E*)-2-cyclopentyl-5-styryl-1,3-benzenediol (305.4 mg).

(*E*)-2-cyclopentyl-5-styryl-1,3-benzenediol was purified by column chromatography (mobile phase: petroleum ether:ethyl acetate = 30:1). The mobile phase was concentrated to give a purified brown solid product (*E*)-2-cyclopentyl-5-styryl-1,3-benzenediol (145.1 mg). Yield: 39.6%.

¹H NMR (400 MHz, DMSO-*d*₆) *δ* 9.10 (s, 2H), 7.57 (d, *J =* 8.0 Hz, 2H), 7.36 (t, *J =* 8.0 Hz, 2H), 7.25 (t, *J =* 8.0 Hz, 1H), 7.01 (d, *J =* 16.0 Hz, 1H), 6.89 (d, *J =* 16.0 Hz, 1H), 3.51-3.42 (m, 1H), 2.02-1.95 (m, 2H), 1.82-1.75 (m, 2H), 1.66-1.55 (m, 4H). ESI-MS *m*/*z* 279.1 [M-H]⁻.

### Example 4

### Synthesis of (E)-2-cyclopentyl-5-(4-fluorostyryl)-1,3-benzenediol

### (1) Synthesis of (E)-2-cyclopentyl-5-(4-fluorostyryl)-1,3-dimethoxybenzene

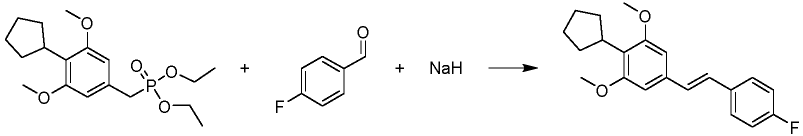

Referring to the synthesis of (*E*)-2-cyclopentyl-1,3-dimethoxy-5-styrylbenzene in **Example 3,** starting materials diethyl 4-cyclopentyl-3,5-dimethoxybenzylphosphonate (1.01 g, 2.83 mmol) and *p*-fluorobenzaldehyde (525.7 mg, 4.24 mmol) were used for reaction, and the reaction solution was purified to give a product (*E*)-2-cyclopentyl-5-(4-fluorostyryl)-1,3-dimethoxybenzene (252 mg). Yield: 27.2%.

### (2) Synthesis of (E)-2-cyclopentyl-5-(4-fluorostyryl)-1,3-benzenediol

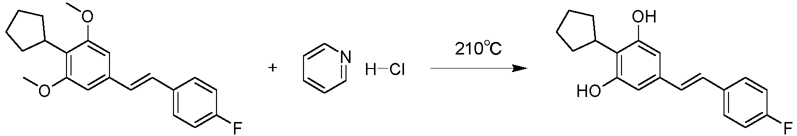

Referring to the synthesis of (*E*)-2-cyclopentyl-5-styryl-1,3-benzenediol in **Example 3,** a starting material (*E*)-2-cyclopentyl-5-(4-fluorostyryl)-1,3-dimethoxybenzene (252.5 mg, 0.77 mmol) was used for reaction, and the reaction solution was purified to give a product (*E*)-2-cyclopentyl-5-(4-fluorostyryl)-1,3-benzenediol (103.3 mg). Yield: 44.8%.

¹H NMR (400 MHz, DMSO-*d*₆) *δ* 9.09 (s, 2H), 7.64-7.61 (m, 2H), 7.21-7.16 (m, 2H), 6.97 (d, *J* = 16.0 Hz, 1H), 6.88 (d, *J* = 16.0 Hz, 1H), 6.47 (s, 2H), 3.50-3.41 (m, 1H), 1.99-1.96 (m, 2H), 1.80-1.75 (m, 2H), 1.64-1.55 (m,4H). ESI-MS *m*/*z* 297.1 [M-H]⁻.

### Example 5

### Synthesis of (E)-2-cyclopentyl-5-(4-chlorostyryl)-1,3-benzenediol

### (1) Synthesis of (E)-2-cyclopentyl-5-(4-chlorostyryl)-1,3-dimethoxybenzene

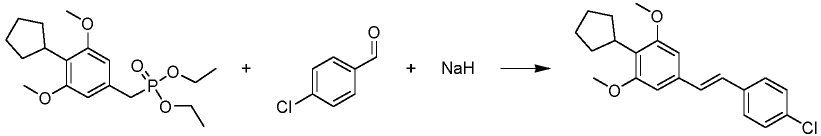

Referring to the synthesis of (*E*)-2-cyclopentyl-1,3-dimethoxy-5-styrylbenzene in **Example 3,** starting materials diethyl 4-cyclopentyl-3,5-dimethoxybenzylphosphonate (1.01 g, 2.83 mmol) and *p*-chlorobenzaldehyde (593.6 mg, 4.24 mmol) were used for reaction, and the reaction solution was purified to give a product (*E*)-2-cyclopentyl-5-(4-chlorostyryl)-1,3-dimethoxybenzene (451.3 mg). Yield: 46.5%.

### (2) Synthesis of (E)-2-cyclopentyl-5-(4-chlorostyryl)-1,3-benzenediol

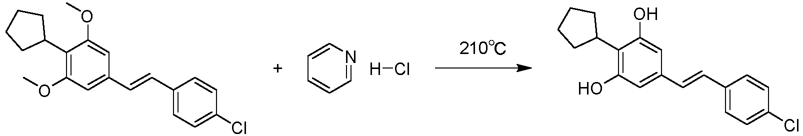

Referring to the synthesis of (*E*)-2-cyclopentyl-5-styryl-1,3-benzenediol in **Example 3,** a starting material (*E*)-2-cyclopentyl-5-(4-chlorostyryl)-1,3-dimethoxybenzene (451.3 mg, 1.32 mmol) was used for reaction, and the reaction solution was purified to give a product (*E*)-2-cyclopentyl-5-(4-chlorostyryl)-1,3-benzenediol (251.3 mg). Yield: 60.6%.

¹H NMR (400 MHz, DMSO-*d*₆) *δ* 9.11 (s, 2H), 7.61 (d, *J* = 8.0 Hz, 2H), 7.40 (d, *J* = 8.0 Hz, 2H), 7.04 (d, *J =* 16.0 Hz, 1H), 7.88 (d, *J* = 16.0 Hz, 1H), 6.49 (s, 2H), 3.51-3.42 (m, 1H), 2.00-1.95 (m, 2H), 1.80-1.75 (m, 2H), 1.64-1.55 (m, 4H). ESI-MS *m*/*z* 313.1 [M-H]⁻.

### Example 6

### Synthesis of (E)-2-cyclopentyl-5-(4-bromostyryl)-1,3-benzenediol

### (1) Synthesis of (E)-2-cyclopentyl-5-(4-bromostyryl)-1,3-dimethoxybenzene

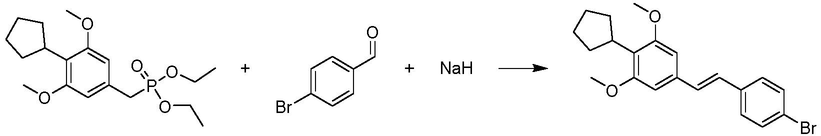

Referring to the synthesis of (*E*)-2-cyclopentyl-1,3-dimethoxy-5-styrylbenzene in **Example 3,** starting materials diethyl 4-cyclopentyl-3,5-dimethoxybenzylphosphonate (1.01 g, 2.83 mmol) and *p*-bromobenzaldehyde (784.4 mg, 4.24 mmol) were used for reaction, and the reaction solution was purified to give a product (*E*)-2-cyclopentyl-5-(4-bromostyryl)-1,3-dimethoxybenzene (521.7 mg). Yield: 47.4%.

### (2) Synthesis of (E)-2-cyclopentyl-5-(4-bromostyryl)-1,3-benzenediol

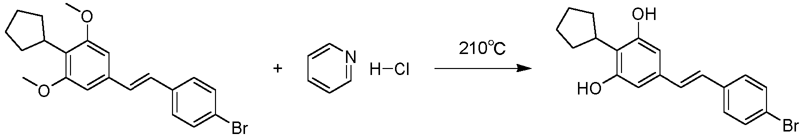

Referring to the synthesis of (*E*)-2-cyclopentyl-5-styryl-1,3-benzenediol in **Example 3,** a starting material (*E*)-2-cyclopentyl-5-(4-bromostyryl)-1,3-dimethoxybenzene (521.7 mg, 1.35 mmol) was used for reaction, and the reaction solution was purified to give a product (*E*)-2-cyclopentyl-5-(4-bromostyryl)-1,3-benzenediol (215.2 mg). Yield: 44.5%.

¹H NMR (400 MHz, DMSO-*d*₆) *δ* 9.10 (s, 2H), 7.53 (s, 2H), 7.05 (d, *J* = 16.0 Hz, 4H), 6.85 (d, *J* = 16.0 Hz, 2H), 3.51-3.42(m, 1H), 1.99 -1.95 (m, 2H), 1.81-1.74 (m, 2H), 1.63-1.54 (m, 4H). ESI-MS: *m*/*z* 357.0 [M-H]⁻.

### Example 7

### Synthesis of diethyl 4-cyclohexyl-3,5-dimethoxybenzylphosphonate

### (1) Synthesis of 4-cyclohexyl-3,5-dimethoxybenzoic acid

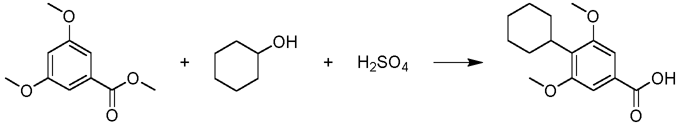

Methyl 3,5-dimethoxybenzoate (20.12 g, 102.55 mmol) and concentrated sulfuric acid (50 mL) were added to a single-neck flask. The mixture was stirred for dissolving. In an ice bath, cyclohexanol (25.68 g, 256.37 mmol) was added dropwise to the single-neck flask. After the addition, the ice bath was removed, and the reaction system was slowly warmed to 70 °C and incubated for 4 h. The reaction was monitored by TLC and stopped after the depletion of the starting materials was confirmed. The reaction solution was cooled to room temperature and poured into a beaker. Saturated sodium bicarbonate solution was added to the beaker to adjust the reaction solution to pH 3-5. Three extractions were performed with ethyl acetate (200 mL) and water (200 mL). The ethyl acetate phases were combined, washed with saturated sodium chloride solution (100 mL), and dried over anhydrous sodium sulfate. After drying, ethyl acetate was removed by concentration at reduced pressure to give a crude brown solid product 4-cyclohexyl-3,5-dimethoxybenzoic acid (27.10 g). Yield: 100.0%.

### (2) Synthesis of methyl 4-cyclohexyl-3,5-dimethoxybenzoate

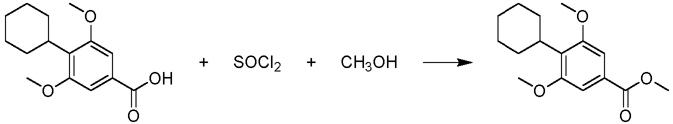

4-Cyclohexyl-3,5-dimethoxybenzoic acid (27.10 g, 102.53 mmol) and methanol (50 mL) were added to a single-neck flask. The mixture was stirred for dissolving. In an ice bath, thionyl chloride (18.29 g, 153.79 mmol) was added dropwise to the single-neck flask, with white smoke observed. After the addition of thionyl chloride, the ice bath was removed, and the reaction system was warmed to 60 °C and incubated for 1 h. The reaction was monitored and stopped after the depletion of the starting materials was confirmed. The reaction solution was cooled to room temperature and concentrated at reduced pressure to remove methanol and excess thionyl chloride. Three extractions were performed with ethyl acetate (150 mL) and water (150 mL). The ethyl acetate phases were combined, washed with saturated sodium chloride solution (100 mL), and dried over anhydrous sodium sulfate for 5 h. After drying, ethyl acetate was removed by concentration at reduced pressure to give a crude black solid product methyl 4-cyclohexyl-3,5-dimethoxybenzoate (28.23 g). Yield: 98.9%.

Methyl 4-cyclohexyl-3,5-dimethoxybenzoate was purified by column chromatography (mobile phase: petroleum ether:ethyl acetate = 15:1). The mobile phase was concentrated to give a purified yellow solid product methyl 4-cyclohexyl-3,5-dimethoxybenzoate (19.51 g). Purification yield: 69.1%.

### (3) Synthesis of 4-cyclohexyl-3,5-dimethoxybenzyl alcohol

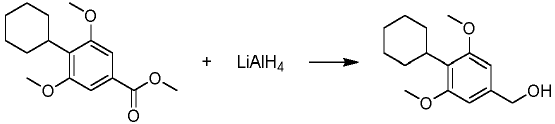

Methyl 4-cyclohexyl-3,5-dimethoxybenzoate (27.10 g, 97.36 mmol) and tetrahydrofuran (271 mL) were added to a single-neck flask. The mixture was stirred for dissolving. In an ice bath, lithium aluminum hydride (7.4 g, 194.73 mmol) was slowly added in batches, and the ice bath was removed after the addition. The reaction solution was warmed to room temperature and incubated for 1 h. The reaction was monitored and stopped after the depletion of the starting materials was confirmed. In an ice bath, water was added to the reaction solution to quench lithium aluminum hydride until the solution became off-white. The mixture was filtered at reduced pressure, and the filtrate was extracted thrice with ethyl acetate (150 mL) and water (150 mL). The ethyl acetate phases were combined, washed with saturated sodium chloride solution (100 mL), and dried over anhydrous sodium sulfate. After drying, ethyl acetate was removed by concentration under reduced pressure to give a crude pale yellow solid product 4-cyclohexyl-3,5-dimethoxybenzyl alcohol (20.23 g). Yield: 83%.

Methyl 4-cyclohexyl-3,5-dimethoxybenzoate was purified by column chromatography (mobile phase: petroleum ether:ethyl acetate = 15:1). The mobile phase was concentrated to give a purified white solid product methyl 4-cyclohexyl-3,5-dimethoxybenzoate (16.13 g). Purification yield: 66.2%.

### (4) Synthesis of 4-cyclohexyl-3,5-dimethoxybenzyl chloride

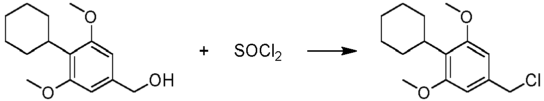

4-Cyclohexyl-3,5-dimethoxybenzyl alcohol (16.13 g, 64.43 mmol) and dichloromethane (30 mL) were added to a single-neck flask. The mixture was stirred for dissolving. **In** an ice bath, thionyl chloride (11.50 g, 96.65 mmol) was added dropwise to the single-neck flask, with white smoke observed. After the addition of thionyl chloride, the ice bath was removed, and the reaction system was incubated at room temperature for 2 h. The reaction was monitored and stopped after the depletion of the starting materials was confirmed. The reaction solution was concentrated at reduced pressure to remove dichloromethane and excess thionyl chloride. Three extractions were performed with ethyl acetate (50 mL) and water (50 mL). The ethyl acetate phases were combined, washed with saturated sodium chloride solution (30 mL), and dried over anhydrous sodium sulfate. After drying, ethyl acetate was removed by concentration at reduced pressure to give a crude black oily product 4-cyclohexyl-3,5-dimethoxybenzyl chloride (15.22 g). Yield: 87.9%.

4-Cyclohexyl-3,5-dimethoxybenzyl chloride was purified by column chromatography (mobile phase: petroleum ether:ethyl acetate = 100:1). The mobile phase was concentrated to give a purified yellow solid product 4-cyclohexyl-3,5-dimethoxybenzyl chloride (11.81 g). Purification yield: 77.6%.

### (5) Synthesis of diethyl 4-cyclohexyl-3,5-dimethoxybenzylphosphonate

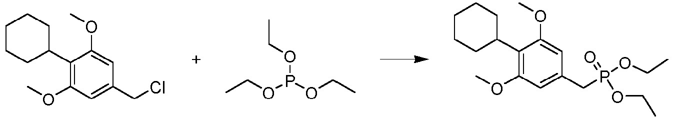

4-Cyclohexyl-3,5-dimethoxybenzyl chloride (11.81 g, 43.94 mmol) was added to a single-neck flask before triethyl phosphite (43.80 g, 263.63 mmol) was added. The mixture was stirred for dissolving. The reaction system was purged with nitrogen thrice, slowly warmed to 160 °C, and incubated for 5 h. The reaction was monitored by TLC and stopped after the depletion of the starting materials was confirmed, and then the reaction solution was cooled to room temperature. After the solvent was removed by concentration at reduced pressure, the crude product was triturated thrice with *n*-hexane (100 mL) to give a pale yellow solid diethyl 4-cyclohexyl-3,5-dimethoxybenzylphosphonate (15.62 g). Yield: 95.9%.

### Example 8

### Synthesis of (E)-2-cyclohexyl-5-styryl-1,3-benzenediol

### (1) Synthesis of (E)-2-cyclohexyl-1,3-dimethoxy-5-styrylbenzene

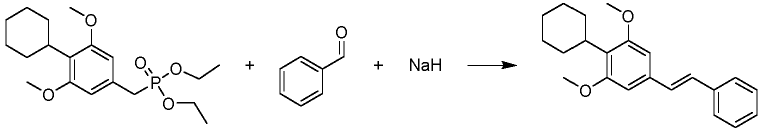

Diethyl 4-cyclohexyl-3,5-dimethoxybenzylphosphonate (1.08 g, 2.92 mmol) and tetrahydrofuran (15 mL) were added to a three-necked flask. The mixture was stirred for dissolving. The reaction system was purged with nitrogen thrice. In an ice bath, 60% sodium hydride (466.5 mg, 11.66 mmol) was added in batches to the three-necked flask, and after the addition, the reaction system was incubated for 1 h. A solution of benzaldehyde (4.37 mmol) in tetrahydrofuran (464.1 mg of benzaldehyde in 10 mL of tetrahydrofuran) was slowly and dropwise added to the three-necked flask. After the addition, the reaction system was slowly warmed to 70 °C and incubated for 4 h. The reaction was monitored by TLC and stopped after the depletion of the starting materials was confirmed. In an ice bath, water was added to the three-necked flask to quench the reaction until no gas was produced, and tetrahydrofuran was removed by concentration at reduced pressure. Three extractions were performed with ethyl acetate (30 mL) and water (30 mL). The ethyl acetate phases were combined, washed with saturated sodium chloride solution (20 mL), and dried over anhydrous sodium sulfate. After drying, ethyl acetate was removed by concentration at reduced pressure to give a crude yellow oily product (*E*)-2-cyclohexyl-1,3-dimethoxy-5-styrylbenzene (625.2 mg).

(*E*)-2-Cyclohexyl-1,3-dimethoxy-5-styrylbenzene was purified by column chromatography (mobile phase: petroleum ether:ethyl acetate = 50:1). The mobile phase was concentrated to give a purified yellow solid product (*E*)-2-cyclohexyl-1,3-dimethoxy-5-styrylbenzene (451.1 mg). Yield: 48.0%.

### (2) Synthesis of (E)-2-cyclohexyl-5-styryl-1,3-benzenediol

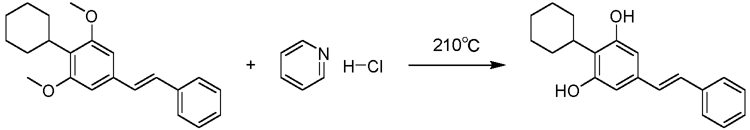

(*E*)-2-cyclohexyl-1,3-dimethoxy-5-styrylbenzene (451.1 mg, 1.40 mmol) was added to a single-neck flask before pyridine hydrochloride (3.23 g, 27.98 mmol) was added. The reaction system was purged with nitrogen thrice, slowly warmed to 210 °C, and incubated for 4 h after pyridine hydrochloride was melted. The reaction was monitored by TLC and stopped after the depletion of the starting materials was confirmed. The reaction solution was cooled to room temperature to solidify and extracted thrice with ethyl acetate (30 mL) and water (30 mL). The ethyl acetate phases were combined, washed with saturated sodium chloride solution (20 mL), and dried over anhydrous sodium sulfate. After drying, ethyl acetate was removed by concentration at reduced pressure to give a crude black solid product (*E*)-2-cyclohexyl-5-styryl-1,3-benzenediol (215.9 mg).

(*E*)-2-cyclohexyl-5-styryl-1,3-benzenediol was purified by column chromatography (mobile phase: petroleum ether:ethyl acetate = 30:1). The mobile phase was concentrated to give a purified brown solid product (*E*)-2-cyclohexyl-5-styryl-1,3-benzenediol (109.3 mg). Yield: 26.5%.

¹H NMR (400 MHz, DMSO-*d*₆) *δ* 9.04 (s, 2H), 7.56 (d, *J =* 8.0 Hz, 2H), 7.35 (t, *J=* 8.0 Hz, 2H), 7.24 (t, *J* = 8.0 Hz, 1H), 7.00 (d, *J* = 16.0 Hz, 1H), 6.87 (d, *J* = 16.0 Hz, 1H), 6.5 (s, 2H), 3.08-3.02 (m, 1H), 2.14-2.06 (m, 2H), 1.75-1.65 (m, 3H), 1.44-1.41 (m, 2H), 1.30-1.23 (m, 3H). ESI-MS *m*/*z* 293.0 [M-H]⁻.

### Example 9

### Synthesis of (E)-2-cyclohexyl-5-(4-fluorostyryl)-1,3-benzenediol

### (1) Synthesis of (E)-2-cyclohexyl-5-(4-fluorostyryl)-1,3-dimethoxybenzene

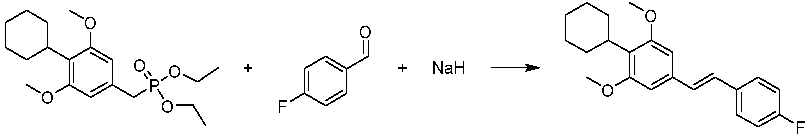

Referring to the synthesis of (*E*)-2-cyclohexyl-1,3-dimethoxy-5-styrylbenzene in Example 8, starting materials diethyl 4-cyclohexyl-3,5-dimethoxybenzylphosphonate (1.08 g, 2.92 mmol) and *p-*fluorobenzaldehyde (525.7 mg, 4.24 mmol) were used for reaction, and the reaction solution was purified to give a product (*E*)-2-cyclohexyl-5-(4-fluorostyryl)-1,3-dimethoxybenzene (483 mg). Yield: 48.7%.

### (2) Synthesis of (E)-2-cyclohexyl-5-(4-fluorostyryl)-1,3-benzenediol

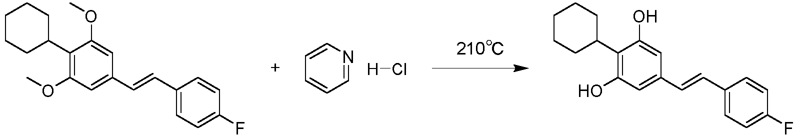

Referring to the synthesis of (*E*)-2-cyclohexyl-5-styryl-1,3-benzenediol in Example 8, a starting material (*E*)-2-cyclohexyl-5-(4-fluorostyryl)-1,3-dimethoxybenzene (483.7 mg, 1.42 mmol) was used for reaction, and the reaction solution was purified to give a product (E)-2-cyclohexyl-5-(4-fluorostyryl)-1,3-benzenediol (171.8 mg). Yield: 38.7%.

¹H NMR (400 MHz, DMSO-*d*₆) *δ* 9.06 (s, 2H), 7.64-7.61 (m, 2H), 7.20-7.17 (m, 2H), 6.96 (d, *J* = 16.0 Hz, 1H), 6.87 (d, *J* = 16.0 Hz, 1H), 6.46 (s, 2H), 3.08-3.02 (m, 1H), 2.15-2.06 (m, 2H), 1.76-1.65 (m, 3H),1.44-1.41 (m, 2H), 1.32-1.16 (m, 3H). ESI-MS *m*/*z* 311.1 [M-H]⁻.

### Example 10

### Synthesis of (E)-2-cyclohexyl-5-(4-chlorostyryl)-1,3-benzenediol

### (1) Synthesis of (E)-2-cyclohexyl-5-(4-chlorostyryl)-1,3-dimethoxybenzene

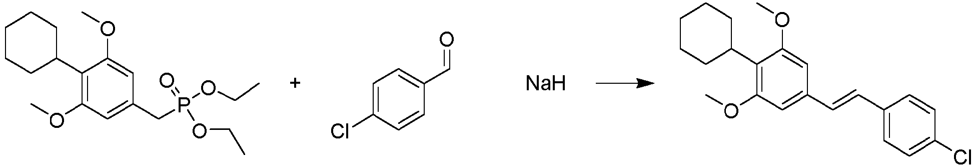

Referring to the synthesis of (*E*)-2-cyclohexyl-1,3-dimethoxy-5-styrylbenzene in Example 8, starting materials diethyl 4-cyclohexyl-3,5-dimethoxybenzylphosphonate (1.08 g, 2.92 mmol) and *p-*chlorobenzaldehyde (593.6 mg, 4.24 mmol) were used for reaction, and the reaction solution was purified to give a product (*E*)-2-cyclohexyl-5-(4-chlorostyryl)-1,3-dimethoxybenzene (475.0 mg). Yield: 45.6%.

### (2) Synthesis of (E)-2-cyclohexyl-5-(4-chlorostyryl)-1,3-benzenediol

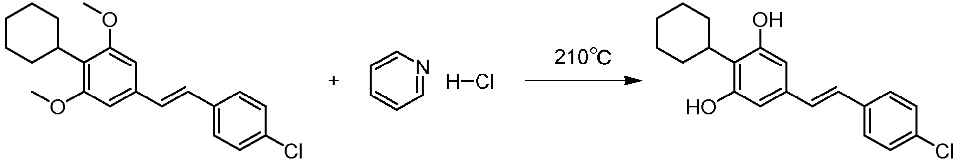

Referring to the synthesis of (*E*)-2-cyclohexyl-5-styryl-1,3-benzenediol in Example 8, a starting material (*E*)-2-cyclohexyl-5-(4-chlorostyryl)-1,3-dimethoxybenzene (475 mg, 1.33 mmol) was used for reaction, and the reaction solution was purified to give a product (*E*)-2-cyclohexyl-5-(4-chlorostyryl)-1,3-benzenediol (163.6 mg). Yield: 37.5%.

¹H NMR (400 MHz, DMSO-*d*₆) *δ* 9.07 (s, 2H), 7.60 (d, *J =* 8.0 Hz, 2H), 7.40 (d, *J =* 8.0 Hz, 2H), 7.03 (d, *J =* 16.0 Hz, 1H), 6.87 (d, *J =* 16.0 Hz, 1H), 6.47 (s, 2H), 3.08 -3.02 (m, 1H), 2.15-2.06 (m, 4H), 1.76-1.65 (m, 4H), 1.44-1.41 (m, 2H). ESI-MS *m*/*z* 327.0 [M-H]⁻.

### Example 11

### Synthesis of (E)-2-cyclohexyl-5-(4-bromostyryl)-1,3-benzenediol

### (1) Synthesis of (E)-2-cyclohexyl-5-(4-bromostyryl)-1,3-dimethoxybenzene

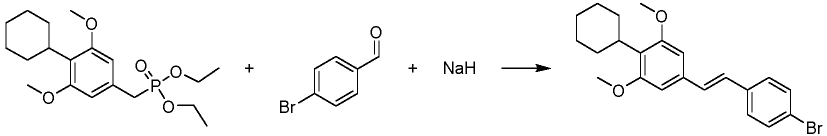

Referring to the synthesis of (*E*)-2-cyclohexyl-1,3-dimethoxy-5-styrylbenzene in Example 8, starting materials diethyl 4-cyclohexyl-3,5-dimethoxybenzylphosphonate (1.08 g, 2.92 mmol) and *p-*bromobenzaldehyde (784.4 mg, 4.24 mmol) were used for reaction, and the reaction solution was purified to give a product (*E*)-2-cyclohexyl-5-(4-bromostyryl)-1,3-dimethoxybenzene (625.3 mg). Yield: 53.4%.

### (2) Synthesis of (E)-2-cyclohexyl-5-(4-bromostyryl)-1,3-benzenediol

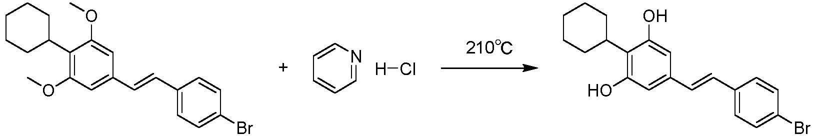

Referring to the synthesis of (*E*)-2-cyclohexyl-5-styryl-1,3-benzenediol in Example 8, a starting material (*E*)-2-cyclohexyl-5-(4-bromostyryl)-1,3-dimethoxybenzene (625.3 mg, 1.56 mmol) was used for reaction, and the reaction solution was purified to give a product (*E*)-2-cyclohexyl-5-(4-bromostyryl)-1,3-benzenediol (194.0 mg). Yield: 33.4%.

¹H NMR (400 MHz, DMSO-*d*₆) *δ* 9.06 (s, 2H), 7.53 (s, 4H), 7.04 (d, *J* = 16.0 Hz, 1H), 6.85 (d, *J* = 16.0 Hz, 1H), 6.47 (s, 2H), 3.08-3.02 (m, 1H), 2.15-2.06 (m, 2H), 1.75-1.65 (m, 3H), 1.44-1.40 (m, 2H), 1.32-1.19 (m, 3H). ESI-MS *m*/*z* 371.0 [M-H]⁻.

### Example 12

### Synthesis of (E)-2-cyclohexyl-5-(3-fluorostyryl)-1,3-benzenediol

### (1) Synthesis of (E)-2-cyclohexyl-5-(3-fluorostyryl)-1,3-dimethoxybenzene

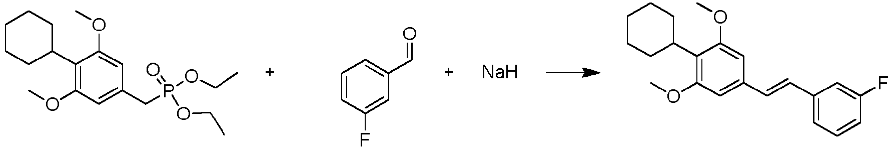

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 3-fluorobenzaldehyde (502.6 mg, 4.05 mmol) were used for reaction, and the reaction solution was purified to give a product (*E*)-2-cyclohexyl-5-(3-fluorostyryl)-1,3-dimethoxybenzene (470.0 mg). Yield: 51.1%.

### (2) Synthesis of (E)-2-cyclohexyl-5-(3-fluorostyryl)-1,3-benzenediol

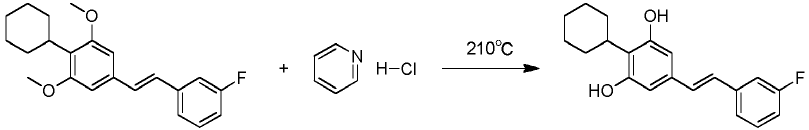

Referring to the synthesis of (*E*)-2-cyclohexyl-5-styryl-1,3-benzenediol in **Example 8,** a starting material (*E*)-2-cyclohexyl-5-(3-fluorostyryl)-1,3-dimethoxybenzene (470 mg, 1.38 mmol) was used for reaction, and the reaction solution was purified to give a product (*E*)-2-cyclohexyl-5-(3-fluorostyryl)-1,3-benzenediol (150 mg). Yield: 34.8%.

¹H NMR (400 MHz, DMSO-*d*₆) *δ* 9.08 (s, 2H), 7.80 (t, *J* = 7.5 Hz, 1H), 7.34 - 7.27 (m, 1H), 7.21 (dd, *J =* 5.7 Hz, 2H), 7.04 (dd, *J* = 16.4 Hz, 2H), 6.50 (s, 2H), 3.06 (t, *J* = 11.5 Hz, 1H), 2.10 (dd, *J* = 11.5 Hz, 2H), 1.70 (dd, *J =* 9.2 Hz, 3H), 1.43 (d, *J =* 11.6 Hz, 2H), 1.36 - 1.12 (m, 3H). ESI-MS *m*/*z* 311.1 [M-H]⁻.

### Example 13

### Synthesis of (E)-2-cyclohexyl-5-(2-fluorostyryl)-1,3-benzenediol

### (1) Synthesis of (E)-2-cyclohexyl-5-(2-fluorostyryl)-1,3-dimethoxybenzene

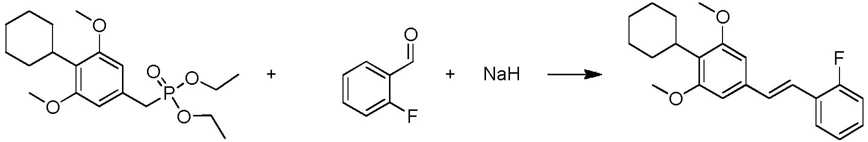

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 2-fluorobenzaldehyde (502.6 mg, 4.05 mmol) were used for reaction, and the reaction solution was purified to give a product (*E*)-2-cyclohexyl-5-(2-fluorostyryl)-1,3-dimethoxybenzene (450 mg). Yield: 49.0%.

### (2) Synthesis of (E)-2-cyclohexyl-5-(2-fluorostyryl)-1,3-benzenediol

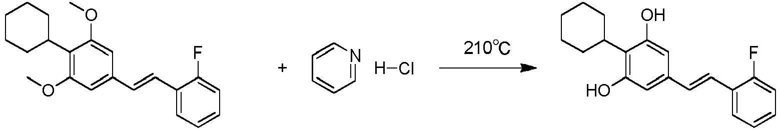

Referring to the synthesis of (*E*)-2-cyclohexyl-5-styryl-1,3-benzenediol in **Example 8,** a starting material (*E*)-2-cyclohexyl-5-(2-fluorostyryl)-1,3-dimethoxybenzene (450 mg. 1.32 mmol) was used for reaction, and the reaction solution was purified to give a product (*E*)-2-cyclohexyl-5-(2-fluorostyryl)-1,3-benzenediol (170 mg). Yield: 41.2%.

¹H NMR (400 MHz, DMSO-*d*₆) *δ* 9.03 (s, 2H), 7.61 (dd, *J* = 5.6 Hz, 2H), 7.18 (t, *J* = 8.8 Hz, 2H), 6.91 (q, J = 16.3 Hz, 2H), 6.46 (s, 2H), 3.05 (t, *J =* 12.1 Hz, 1H), 2.10 (dd, 12.1 Hz, 2H), 1.70 (dd, *J* = 10.1 Hz, 4H), 1.42 (d, *J* = 12.5 Hz, 2H), 1.34 - 1.17 (m, 2H). ESI-MS *m*/*z* 311.1 [M-H]⁻.

### Example 14

### Synthesis of (E)-2-cyclohexyl-5-(2-chlorostyryl)-1,3-benzenediol

### (1) Synthesis of (E)-2-cyclohexyl-5-(2-chlorostyryl)-1,3-dimethoxybenzene

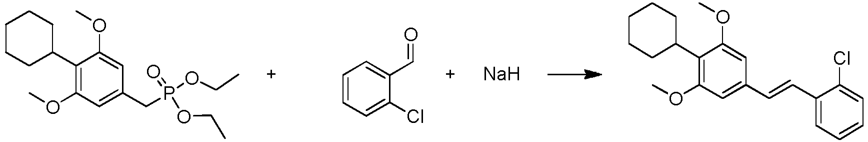

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 2-chlorobenzaldehyde (569.2 mg, 4.05 mmol) were used for reaction, and the reaction solution was purified to give a product (*E*)-2-cyclohexyl-5-(2-chlorostyryl)-1,3-dimethoxybenzene (480 mg). Yield: 49.8%.

### (2) Synthesis of (E)-2-cyclohexyl-5-(2-chlorostyryl)-1,3-benzenediol

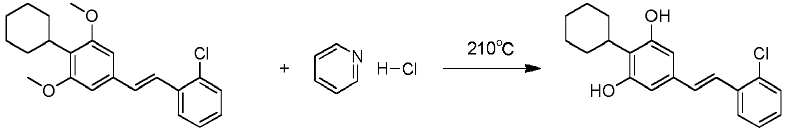

Referring to the synthesis of (*E*)-2-cyclohexyl-5-styryl-1,3-benzenediol in **Example 8,** a starting material (*E*)-2-cyclohexyl-5-(2-chlorostyryl)-1,3-dimethoxybenzene (480 mg, 1.34 mmol) was used for reaction, and the reaction solution was purified to give a product (*E*)-2-cyclohexyl-5-(2-chlorostyryl)-1,3-benzenediol (170 mg). Yield: 38.4%.

¹H NMR (400 MHz, DMSO-*d*₆) *δ* 9.07 (s, 2H), 7.67 (dd, *J* = 5.6 Hz, 2H), 7.53 (t, *J* = 8.8 Hz, 2H), 7.37 (q, *J* = 16.3 Hz, 2H), 7.10 (s, 2H), 3.13 (t, *J =* 12.1 Hz, 1H), 2.26 (dd, 12.1 Hz, 2H), 2.10 (dd, *J* = 10.1 Hz, 4H), 1.72 (d, *J =* 12.5 Hz, 2H), 1.64 - 1.37 (m, 2H). ESI-MS *m*/*z* 327.1 [M-H]⁻.

### Example 15

### Synthesis of (E)-2-cyclohexyl-5-(3-chlorostyryl)-1,3-benzenediol

### (1) Synthesis of (E)-2-cyclohexyl-5-(3-chlorostyryl)-1,3-dimethoxybenzene

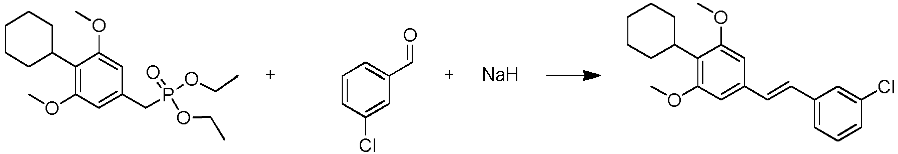

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 3-chlorobenzaldehyde (569.2 mg, 4.05 mmol) were used for reaction, and the reaction solution was purified to give a product (*E*)-2-cyclohexyl-5-(3-chlorostyryl)-1,3-dimethoxybenzene (475 mg). Yield: 49.3%.

### (2) Synthesis of (E)-2-cyclohexyl-5-(3-chlorostyryl)-1,3-benzenediol

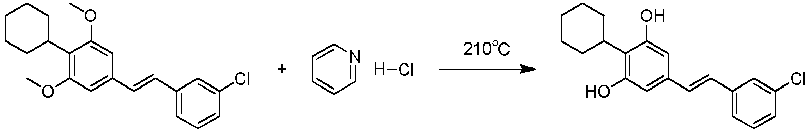

Referring to the synthesis of (*E*)-2-cyclohexyl-5-styryl-1,3-benzenediol in **Example 8,** a starting material (*E*)-2-cyclohexyl-5-(3-chlorostyryl)-1,3-dimethoxybenzene (475 mg, 1.33 mmol) was used for reaction, and the reaction solution was purified to give a product (*E*)-2-cyclohexyl-5-(3-chlorostyryl)-1,3-benzenediol (170 mg). Yield: 38.8%.

¹H NMR (400 MHz, DMSO-*d*₆) *δ* 9.05 (s, 2H), 7.60 (d, *J* = 8.5 Hz, 2H), 7.40 (d, *J* = 8.5 Hz, 2H), 7.03 (d, *J* = 16.3 Hz, 1H), 6.86 (d, *J =* 16.3 Hz, 2H), 6.47 (s, 1H), 3.05 (t, *J =* 12.2 Hz, 1H), 2.10 (dd, *J* =11.0 Hz, 2H), 1.70 (dd, *J =* 10.3 Hz, 2H), 1.43 (d, *J* = 11.7 Hz, 2H), 1.20 (d, *J =* 13.6 Hz, 4H). ESI-MS *m*/*z* 327.1 [M-H]⁻.

### Example 16

### Synthesis of (E)-2-cyclohexyl-5-(2,4-difluorostyryl)-1,3-benzenediol

### (1) Synthesis of (E)-2-cyclohexyl-5-(2,4-difluorostyryl)-1,3-dimethoxybenzene

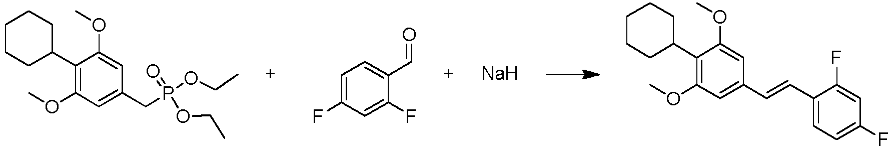

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 2,4-difluorobenzaldehyde (575.4 mg, 4.05 mmol) were used for reaction, and the reaction solution was purified to give a product (*E*)-2-cyclohexyl-5-(2,4-difluorostyryl)-1,3-dimethoxybenzene (470 mg). Yield: 48.6%.

### (2) Synthesis of (E)-2-cyclohexyl-5-(2,4-difluorostyryl)-1,3-benzenediol

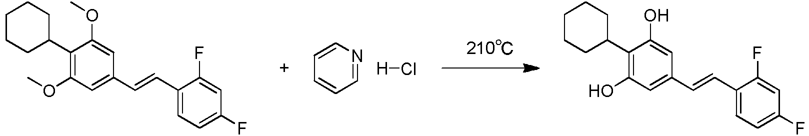

Referring to the synthesis of (*E*)-2-cyclohexyl-5-styryl-1,3-benzenediol in **Example 8,** a starting material (*E*)-2-cyclohexyl-5-(2,4-difluorostyryl)-1,3-dimethoxybenzene (470 mg, 1.31 mmol) was used for reaction, and the reaction solution was purified to give a product (*E*)-2-cyclohexyl-5-(2,4-difluorostyryl)-1,3-benzenediol (170 mg). Yield: 39.2%.

¹H NMR (400 MHz, DMSO-*d*₆) *δ* 9.10 (s, 2H), 7.74 - 7.69 (m, 1H), 7.27 (td, *J* = 9.6, 4.7 Hz, 1H), 7.20 (d, *J* = 16.4 Hz, 1H), 7.15 (dt, *J =* 11.8,Hz, 1H), 6.98 (d, *J =* 16.4 Hz, 1H), 6.50 (s, 2H), 3.08 (t, *J =* 12.1 Hz, 1H), 2.20 - 2.02 (m, 2H), 1.73 (dd, *J* = 31.4, 10.5 Hz, 3H), 1.40 (d, *J =* 12.2 Hz, 2H), 1.25 (t, *J* = 13.4 Hz, 3H). ESI-MS *m*/*z* 329.1 [M-H]⁻.

### Example 17

### Synthesis of (E)-2-cyclohexyl-5-(2,6-difluorostyryl)-1,3-benzenediol

### (1) Synthesis of (E)-2-cyclohexyl-5-(2,6-difluorostyryl)-1,3-dimethoxybenzene

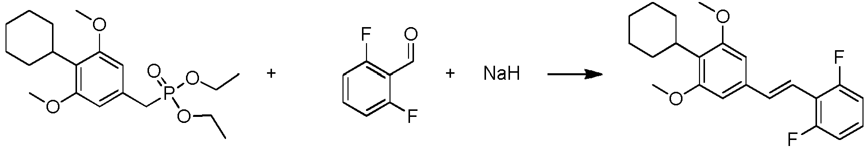

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 2,6-difluorobenzaldehyde (575.4 mg, 4.05 mmol) were used for reaction, and the reaction solution was purified to give a product (*E*)-2-cyclohexyl-5-(2,6-difluorostyryl)-1,3-dimethoxybenzene (450 mg). Yield: 46.5%.

### (2) Synthesis of (E)-2-cyclohexyl-5-(2,6-difluorostyryl)-1,3-benzenediol

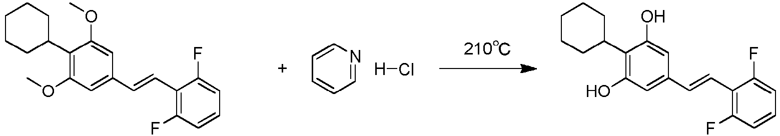

Referring to the synthesis of (*E*)-2-cyclohexyl-5-styryl-1,3-benzenediol in **Example 8,** a starting material (*E*)-2-cyclohexyl-5-(2,6-difluorostyryl)-1,3-dimethoxybenzene (450 mg, 1.26 mmol) was used for reaction, and the reaction solution was purified to give a product (*E*)-2-cyclohexyl-5-(2,6-difluorostyryl)-1,3-benzenediol (150 mg). Yield: 36.2%.

¹H NMR (400 MHz, DMSO-*d*₆) *δ* 9.10 (s, 2H), 7.35 (d, *J* = 7.3 Hz, 2H), 7.17 (d, *J* = 16.3 Hz, 1H), 7.07 (t, *J =* 9.2 Hz, 1H), 6.86 (d, *J =* 16.3 Hz, 1H), 6.48 (s, 2H), 3.06 (t, *J =* 12.0 Hz, 1H), 2.10 (dd, *J* = 11.2 Hz, 2H), 1.70 (dd, *J =* 10.0 Hz, 3H), 1.43 (d, *J =* 11.9 Hz, 2H), 1.27 (dd, *J* =10.6 Hz, 3H). ESI-MS *m*/*z* 329.1 [M-H]⁻.

### Example 18

### Synthesis of (E)-2-cyclohexyl-5-(2,5-difluorostyryl)-1,3-benzenediol

### (1) Synthesis of (E)-2-cyclohexyl-5-(2,5-difluorostyryl)-1,3-dimethoxybenzene

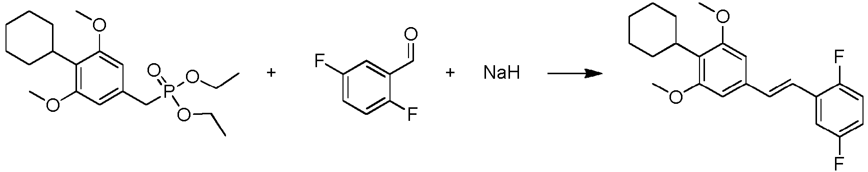

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 2,5-difluorobenzaldehyde (575.4 mg, 4.05 mmol) were used for reaction, and the reaction solution was purified to give a product (*E*)-2-cyclohexyl-5-(2,5-difluorostyryl)-1,3-dimethoxybenzene (445 mg). Yield: 46.0%.

### (2) Synthesis of (E)-2-cyclohexyl-5-(2,5-difluorostyryl)-1,3-benzenediol

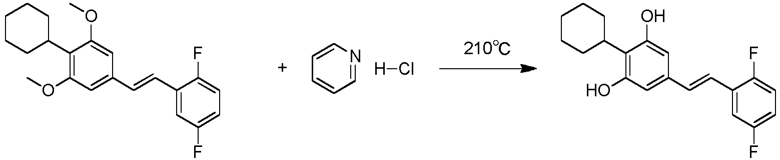

Referring to the synthesis of (*E*)-2-cyclohexyl-5-styryl-1,3-benzenediol in **Example 8,** a starting material (*E*)-2-cyclohexyl-5-(2,5-difluorostyryl)-1,3-dimethoxybenzene (445 mg, 1.24 mmol) was used for reaction, and the reaction solution was purified to give a product (*E*)-2-cyclohexyl-5-(2,5-difluorostyryl)-1,3-benzenediol (168 mg). Yield: 41.0%.

¹H NMR (400 MHz, DMSO-*d*₆) *δ* 9.06 (s, 2H), 7.86 (dd, *J* = 7.8 Hz, 1H), 7.30 - 7.23 (m, 1H), 7.12 (d, *J =* 8.4 Hz, 1H), 7.05 (d, *J =* 16.4 Hz, 1H), 6.93 (d, *J* = 16.5 Hz, 1H), 6.48 (s, 2H), 3.11 - 2.98 (m, 1H), 2.10 (dd, *J =* 12.1 Hz, 2H), 1.70 (dd, *J =* 8.4 Hz, 3H), 1.43 (d, *J =* 11.3 Hz, 2H), 1.19 (dd, *J* =10.7 Hz, 3H). ESI-MS *m*/*z* 329.1 [M-H]⁻.

### Example 19

### Synthesis of (E)-2-cyclohexyl-5-(3,4-difluorostyryl)-1,3-benzenediol

### (1) Synthesis of (E)-2-cyclohexyl-5-(3,4-difluorostyryl)-1,3-dimethoxybenzene

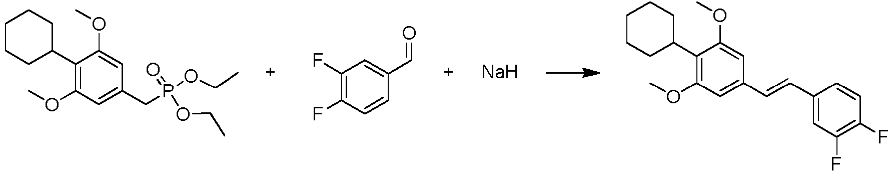

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 3,4-difluorobenzaldehyde (575.4 mg, 4.05 mmol) were used for reaction, and the reaction solution was purified to give a product (*E*)-2-cyclohexyl-5-(3,4-difluorostyryl)-1,3-dimethoxybenzene (463 mg). Yield: 47.9%.

### (2) Synthesis of (E)-2-cyclohexyl-5-(3,4-difluorostyryl)-1,3-benzenediol

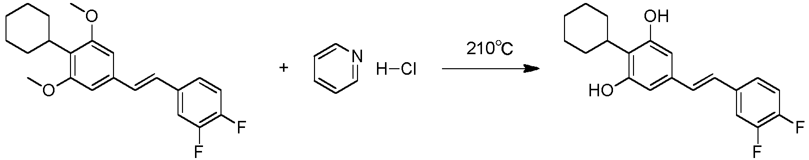

Referring to the synthesis of (E)-2-cyclohexyl-5-styryl-1,3-benzenediol in **Example 8,** a starting material (*E*)-2-cyclohexyl-5-(3,4-difluorostyryl)-1,3-dimethoxybenzene (463 mg, 1.29 mmol) was used for reaction, and the reaction solution was purified to give a product (*E*)-2-cyclohexyl-5-(3,4-difluorostyryl)-1,3-benzenediol (168 mg). Yield: 39.4%.

¹H NMR (400 MHz, DMSO-*d*₆) *δ* 9.11 (s, 2H), 7.77 - 7.68 (m, 1H), 7.27 (td, *J* = 9.6Hz, 1H), 7.19 (d, *J =* 16.4 Hz, 1H), 7.12 (dt, *J* = 11.8,Hz, 1H), 6.95 (d, *J* = 16.4 Hz, 1H), 6.52 (s, 2H), 3.07 (t, *J* = 12.1 Hz, 1H), 2.20 - 2.05 (m, 2H), 1.71 (dd, *J* =10.5 Hz, 3H), 1.44 (d, *J* = 12.2 Hz, 2H), 1.27 (t, *J* = 13.4 Hz, 3H). ESI-MS *m*/*z* 329.1 [M-H]⁻.

### Example 20

### Synthesis of (E)-2-cyclohexyl-5-(2,3-difluorostyryl)-1,3-benzenediol

### (1) Synthesis of (E)-2-cyclohexyl-5-(2,3-difluorostyryl)-1,3-dimethoxybenzene

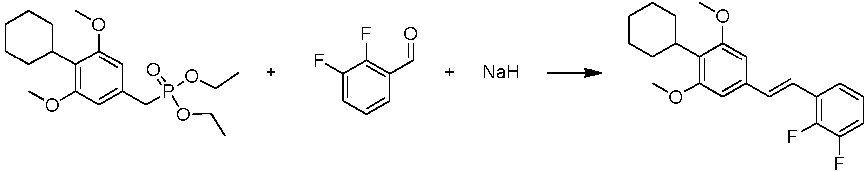

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 2,3-difluorobenzaldehyde (575.4 mg, 4.05 mmol) were used for reaction, and the reaction solution was purified to give a product (*E*)-2-cyclohexyl-5-(2,3-difluorostyryl)-1,3-dimethoxybenzene (468 mg). Yield: 48.4%.

### (2) Synthesis of (E)-2-cyclohexyl-5-(2,3-difluorostyryl)-1,3-benzenediol

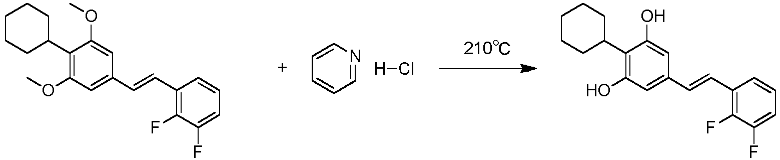

Referring to the synthesis of (*E*)-2-cyclohexyl-5-styryl-1,3-benzenediol in **Example 8,** a starting material (*E*)-2-cyclohexyl-5-(2,3-difluorostyryl)-1,3-dimethoxybenzene (468 mg, 1.31 mmol) was used for reaction, and the reaction solution was purified to give a product (*E*)-2-cyclohexyl-5-(2,3-difluorostyryl)-1,3-benzenediol (173 mg). Yield: 40.1%.

¹H NMR (400 MHz, DMSO-*d*₆) *δ* 9.10 (s, 2H), 7.72 (ddd, *J* = 9.5Hz, 1H), 7.27 (td, *J* = 10.1Hz, 1H), 7.17 (t, *J* = 10.3 Hz, 1H), 7.18 - 7.08 (m, 1H), 6.94 (d, *J* = 16.6 Hz, 1H), 6.50 (s, 2H), 3.06 (t, *J* = 12.1 Hz, 1H), 2.10 (dd, *J =* 11.2 Hz, 2H), 1.70 (dd, *J* =10.0 Hz, 3H), 1.43 (d, *J =* 11.1 Hz, 2H), 1.24 - 1.12 (m, 3H). ESI-MS *m*/*z* 329.1 [M-H]⁻.

### Example 21

### Synthesis of (E)-2-cyclohexyl-5-(3,5-difluorostyryl)-1,3-benzenediol

### (1) Synthesis of (E)-2-cyclohexyl-5-(3,5-difluorostyryl)-1,3-dimethoxybenzene

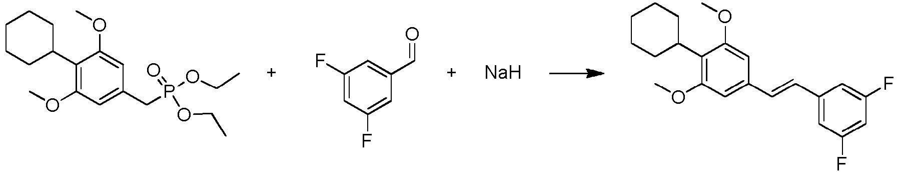

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 3,5-difluorobenzaldehyde (575.4 mg, 4.05 mmol) were used for reaction, and the reaction solution was purified to give a product (*E*)-2-cyclohexyl-5-(3,5-difluorostyryl)-1,3-dimethoxybenzene (470 mg). Yield: 48.6%.

### (2) Synthesis of (E)-2-cyclohexyl-5-(3,5-difluorostyryl)-1,3-benzenediol

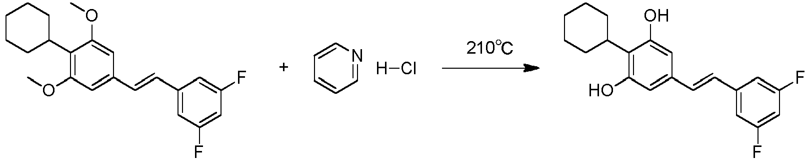

Referring to the synthesis of (*E*)-2-cyclohexyl-5-styryl-1,3-benzenediol in **Example 8,** a starting material (*E*)-2-cyclohexyl-5-(3,5-difluorostyryl)-1,3-dimethoxybenzene (470 mg, 1.31 mmol) was used for reaction, and the reaction solution was purified to give a product (*E*)-2-cyclohexyl-5-(3,5-difluorostyryl)-1,3-benzenediol (165.8 mg). Yield: 38.3%.

¹H NMR (400 MHz, DMSO-*d*₆) *δ* 9.13 (s, 2H), 7.43 - 7.27 (m, 2H), 7.13 (dd, *J* = 18.5, 12.4 Hz, 2H), 6.85 (d, *J* = 16.8 Hz, 2H), 6.48 (s, 1H), 3.06 (t, *J* = 11.7 Hz, 1H), 2.10 (dd, *J* = 13.1 Hz, 2H), 1.70 (dd, *J =* 9.1 Hz, 3H), 1.42 (d, *J =* 12.0 Hz, 2H), 1.35 - 1.20 (t, *J =* 12.0 Hz, 3H). ESI-MS *m*/*z* 329.1 [M-H]⁻.

### Example 22

### Synthesis of (E)-2-cyclohexyl-5-(2,4,5-trifluorostyryl)-1,3-benzenediol

### (1) Synthesis of (E)-2-cyclohexyl-5-(2,4,5-trifluorostyryl)-1,3-dimethoxybenzene

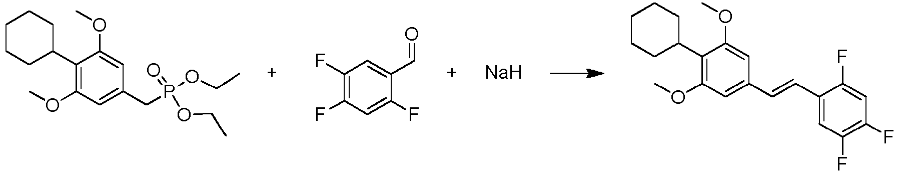

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 2,4,5-trifluorobenzaldehyde (648 mg, 4.04 mmol) were used for reaction, and the reaction solution was purified to give a product (*E*)-2-cyclohexyl-5-(2,4,5-trifluorostyryl)-1,3-dimethoxybenzene (478 mg). Yield: 47.0%.

### (2) Synthesis of (E)-2-cyclohexyl-5-(2,4,5-trifluorostyryl)-1,3-benzenediol

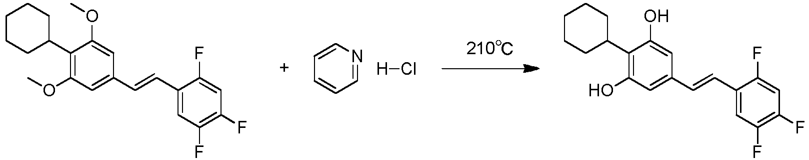

Referring to the synthesis of (*E*)-2-cyclohexyl-5-styryl-1,3-benzenediol in **Example 8,** a starting material (*E*)-2-cyclohexyl-5-(2,4,5-trifluorostyryl)-1,3-dimethoxybenzene (478 mg, 1.27 mmol) was used for reaction, and the reaction solution was purified to give a product (*E*)-2-cyclohexyl-5-(2,4,5-trifluorostyryl)-1,3-benzenediol (163 mg). Yield: 36.8%.

¹H NMR (400 MHz, DMSO-*d*₆) *δ* 9.02 (s, 2H), 7.63 (dd, *J =*7.3 Hz, 2H), 6.88 (dd, *J* = 16.4 Hz, 1H), 6.81 - 6.66 (m,2H), 6.44 (s, 1H), 3.04 (t, J = 9.8 Hz, 1H), 2.09 (dd, *J* = 13.0 Hz, 2H), 1.70 (dd, *J* = 9.6 Hz, 3H), 1.42 (d, J = 11.2 Hz, 2H), 1.34 - 1.22 (m, 3H). ESI-MS *m*/*z* 347.1 [M-H]⁻.

### Example 23

### Synthesis of (E)-2-cyclohexyl-5-(2,3,4-trifluorostyryl)-1,3-benzenediol

### (1) Synthesis of (E)-2-cyclohexyl-5-(2,3,4-trifluorostyryl)-1,3-dimethoxybenzene

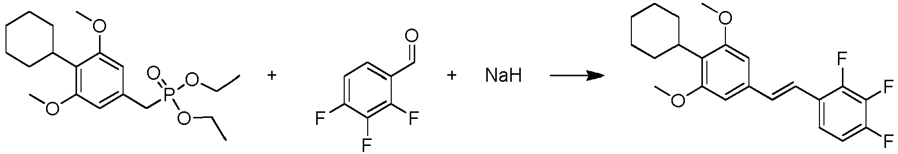

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 2,3,4-trifluorobenzaldehyde (648 mg, 4.04 mmol) were used for reaction, and the reaction solution was purified to give a product (*E*)-2-cyclohexyl-5-(2,3,4-trifluorostyryl)-1,3-dimethoxybenzene (450 mg). Yield: 44.3%.

### (2) Synthesis of (E)-2-cyclohexyl-5-(2,3,4-trifluorostyryl)-1,3-benzenediol

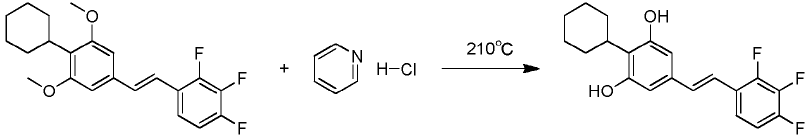

Referring to the synthesis of (*E*)-2-cyclohexyl-5-styryl-1,3-benzenediol in **Example 8,** a starting material (*E*)-2-cyclohexyl-5-(2,3,4-trifluorostyryl)-1,3-dimethoxybenzene (450 mg, 1.20 mmol) was used for reaction, and the reaction solution was purified to give a product (E)-2-cyclohexyl-5-(2,3,4-trifluorostyryl)-1,3-benzenediol (161 mg). Yield: 38.7%.

¹H NMR (400 MHz, DMSO-*d*₆) *δ* 9.09 (s, 2H), 7.28 - 7.12 (m, 2H), 6.84 (d, *J* = 16.2 Hz, 1H), 6.71 (d, *J* = 16.2 Hz, 1H), 6.41 (s, 2H), 4.75 - 4.69 (m, 1H), 3.09 - 2.98 (m, 2H), 2.09 (dd, *J* = 12.2 Hz, 2H), 1.69 (dd, *J* =10.1 Hz, 2H), 1.42 (d, *J* = 12.4 Hz, 2H), 1.33 - 1.15 (m, 2H). ESI-MS *m*/*z* 347.1 [M-H]⁻.

### Example 24

### Synthesis of (E)-2-cyclohexyl-5-(3,4,5-trifluorostyryl)-1,3-benzenediol

### (1) Synthesis of (E)-2-cyclohexyl-5-(3,4,5-trifluorostyryl)-1,3-dimethoxybenzene

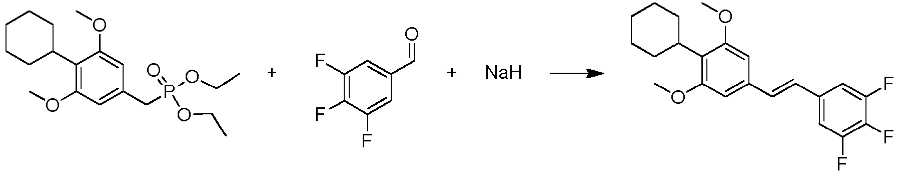

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 3,4,5-trifluorobenzaldehyde (648 mg, 4.04 mmol) were used for reaction, and the reaction solution was purified to give a product (*E*)-2-cyclohexyl-5-(3,4,5-trifluorostyryl)-1,3-dimethoxybenzene (463 mg). Yield: 45.6%.

### (2) Synthesis of (E)-2-cyclohexyl-5-(3,4,5-trifluorostyryl)-1,3-benzenediol

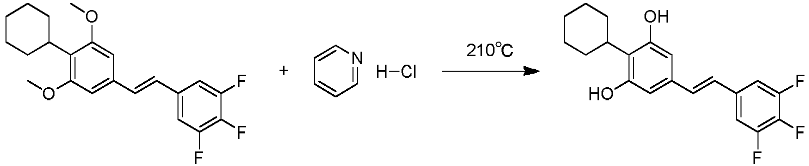

Referring to the synthesis of (*E*)-2-cyclohexyl-5-styryl-1,3-benzenediol in **Example 8,** a starting material (*E*)-2-cyclohexyl-5-(3,4,5-trifluorostyryl)-1,3-dimethoxybenzene (463 mg, 1.23 mmol) was used for reaction, and the reaction solution was purified to give a product (*E*)-2-cyclohexyl-5-(3,4,5-trifluorostyryl)-1,3-benzenediol (157 mg). Yield: 36.6%.

¹H NMR (400 MHz, DMSO-*d*₆) *δ* 10.30 (d, *J* = 16.4 Hz, 1H), 7.55 - 7.31 (m, 1H), 7.32 - 7.17 (m, 1H), 7.17 (s, 1H), 6.96 (t, *J* = 16.2 Hz, 2H), 6.90 - 6.77 (m, 1H), 6.46 (s, 1H), 3.05 (dt, *J =* 11.4, 8.3 Hz, 1H), 2.33 - 1.89 (m, 2H), 1.92 - 1.60 (m, 2H), 1.44 (t, *J =* 12.6 Hz, 1H), 1.42 - 1.19 (m, 5H). ESI-MS *m*/*z* 347.1 [M-H]⁻.

### Example 25

### Synthesis of (E)-2-cyclohexyl-5-(2,4,6-trifluorostyryl)-1,3-benzenediol

### (1) Synthesis of (E)-2-cyclohexyl-5-(2,4,6-trifluorostyryl)-1,3-dimethoxybenzene

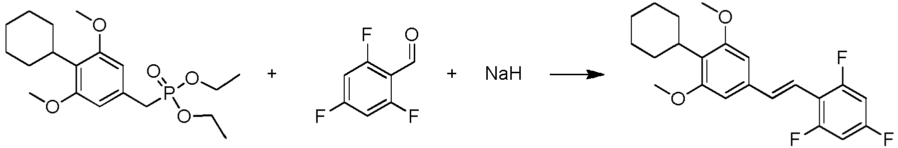

Referring to the synthesis of (*E*)-2-cyclohexyl-1,3-dimethoxy-5-styrylbenzene in **Example 8,** starting materials diethyl 4-cyclohexyl-3,5-dimethoxybenzylphosphonate (1.0 g, 2.70 mmol) and 2,4,6-trifluorobenzaldehyde (648 mg, 4.04 mmol) were used for reaction, and the reaction solution was purified to give a product (*E*)-2-cyclohexyl-5-(2,4,6-trifluorostyryl)-1,3-dimethoxybenzene (453 mg). Yield: 44.6%.

### (2) Synthesis of (E)-2-cyclohexyl-5-(2,4,6-trifluorostyryl)-1,3-benzenediol

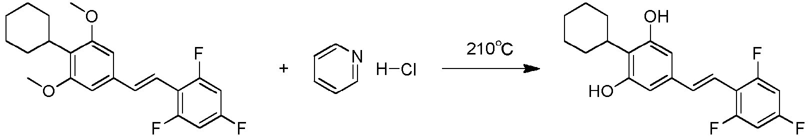

Referring to the synthesis of (*E*)-2-cyclohexyl-5-styryl-1,3-benzenediol in **Example 8,** a starting material (*E*)-2-cyclohexyl-5-(2,4,6-trifluorostyryl)-1,3-dimethoxybenzene (453 mg, 1.20 mmol) was used for reaction, and the reaction solution was purified to give a product (*E*)-2-cyclohexyl-5-(2,4,6-trifluorostyryl)-1,3-benzenediol (158 mg). Yield: 37.7%.

¹H NMR (400 MHz, DMSO-*d*₆) *δ* 9.02 (s, 2H), 7.62 (dd, *J* = 12.3 Hz, 1H), 6.92 (d, *J* = 16.5 Hz, 2H), 6.91 - 6.79 (m, 1H), 6.74 (dd, *J* = 10.7 Hz, 1H), 6.44 (s, 1H), 3.04 (dd, *J* = 10.3 Hz, 1H), 2.09 (dd, *J* =12.3 Hz, 2H), 1.69 (dd, *J =* 8.3 Hz, 2H), 1.42 (d, *J =* 10.4 Hz, 2H), 1.27 (dd, *J =* 8.3 Hz, 4H). ESI-MS *m*/*z* 347.1 [M-H]⁻.

### Example 26

### Synthesis of (E)-2-cyclopentyl-5-(3-fluorostyryl)-1,3-benzenediol

### (1) Synthesis of (E)-2-cyclopentyl-5-(3-fluorostyryl)-1,3-dimethoxybenzene

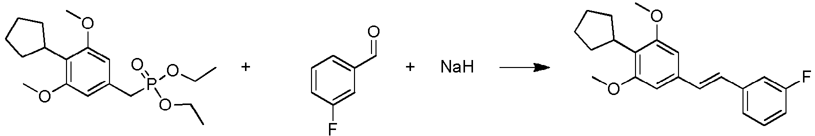

Referring to the synthesis of (*E*)-2-cyclopentyl-1,3-dimethoxy-5-styrylbenzene in **Example 3,** starting materials diethyl 4-cyclopentyl-3,5-dimethoxybenzylphosphonate (1.0 g, 2.81 mmol) and 3-fluorobenzaldehyde (522.4 mg, 4.21 mmol) were used for reaction, and the reaction solution was purified to give a product (*E*)-2-cyclopentyl-5-(3-fluorostyryl)-1,3-dimethoxybenzene (480 mg). Yield: 52.4%.

### (2) Synthesis of (E)-2-cyclopentyl-5-(3-fluorostyryl)-1,3-benzenediol

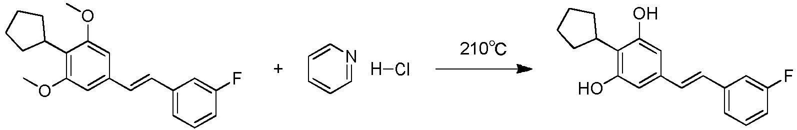

Referring to the synthesis of (*E*)-2-cyclopentyl-5-styryl-1,3-benzenediol in **Example 3,** a starting material (*E*)-2-cyclopentyl-5-(3-fluorostyryl)-1,3-dimethoxybenzene (480 mg, 1.47 mmol) was used for reaction, and the reaction solution was purified to give a product (*E*)-2-cyclopentyl-5-(3-fluorostyryl)-1,3-benzenediol (150 mg). Yield: 34.2%.

¹H NMR (400 MHz, DMSO-*d*₆) *δ* 9.10 (s, 2H), 7.48-7.45 (d, 1H), 7.4-7.38 (d, *J =* 16.0 Hz, 1H), 7.12-7.01 (m, *J* = 16.0 Hz, 2H), 6.91-6.87(d, 2H), 6.55(s, 2H), 2.65-2.59(m,1H),2.33-2.29 (m, 1H), 1.99-1.95(m, 2H), 1.78-1.75 (m, 1H),1.54-1.50 (m, 4H). ESI-MS *m*/*z* 297.1 [M-H]⁻.

### Example 27

### Synthesis of (E)-2-cyclopentyl-5-(2-fluorostyryl)-1,3-benzenediol

### (1) Synthesis of (E)-2-cyclopentyl-5-(2-fluorostyryl)-1,3-dimethoxybenzene

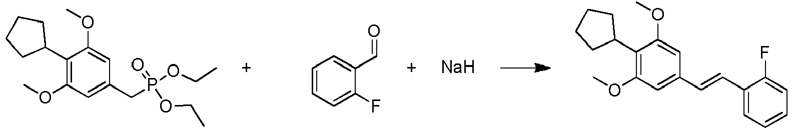

Referring to the synthesis of (*E*)-2-cyclopentyl-1,3-dimethoxy-5-styrylbenzene in **Example 3,** starting materials diethyl 4-cyclopentyl-3,5-dimethoxybenzylphosphonate (1.0 g, 2.81 mmol) and 2-fluorobenzaldehyde (522.4 mg, 4.21 mmol) were used for reaction, and the reaction solution was purified to give a product (*E*)-2-cyclopentyl-5-(2-fluorostyryl)-1,3-dimethoxybenzene (500 mg). Yield: 54.6%.

### (2) Synthesis of (E)-2-cyclopentyl-5-(2-fluorostyryl)-1,3-benzenediol

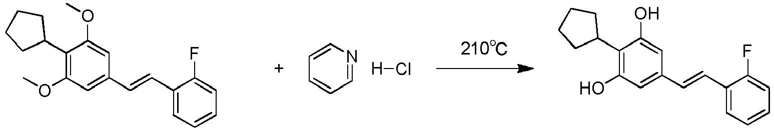

Referring to the synthesis of (*E*)-2-cyclopentyl-5-styryl-1,3-benzenediol in **Example 3,** a starting material (*E*)-2-cyclopentyl-5-(2-fluorostyryl)-1,3-dimethoxybenzene (500 mg, 1.53 mmol) was used for reaction, and the reaction solution was purified to give a product (*E*)-2-cyclopentyl-5-(2-fluorostyryl)-1,3-benzenediol (200 mg). Yield: 43.8%.

¹H NMR (400 MHz, DMSO-*d*₆) *δ* 9.13 (s, 2H), 7.83-7.79 (t, *J* = 16.0 Hz 1H), 7.34-7.28 (t, *J* = 16.0 Hz, 1H), 7.24-7.19 (t, *J =* 16.0 Hz, 2H), 7.13-7.08 (d, 1H), 7.02-6.98 (d, 1H), 6.5 (s, 2H), 3.51-3.42 (m, 1H), 2.0-1.96 (t, 2H), 1.78 (s, 2H),1.64-1.55 (m, 4H). ESI-MS *m*/*z* 297.1 [M-H]⁻.

### Example 28

### Synthesis of (E)-2-cyclopentyl-5-(2-chlorostyryl)-1,3-benzenediol

### (1) Synthesis of (E)-2-cyclopentyl-5-(2-chlorostyryl)-1,3-dimethoxybenzene

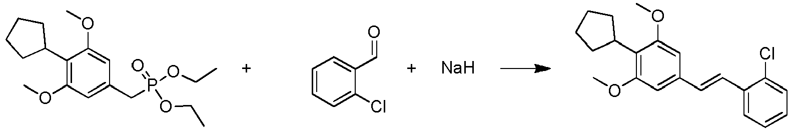

Referring to the synthesis of (*E*)-2-cyclopentyl-1,3-dimethoxy-5-styrylbenzene in **Example 3,** starting materials diethyl 4-cyclopentyl-3,5-dimethoxybenzylphosphonate (1.0 g, 2.81 mmol) and 2-chlorobenzaldehyde (591.6 mg, 4.21 mmol) were used for reaction, and the reaction solution was purified to give a product (*E*)-2-cyclopentyl-5-(2-chlorostyryl)-1,3-dimethoxybenzene (450 mg). Yield: 46.8%.

### (2) Synthesis of (E)-2-cyclopentyl-5-(2-chlorostyryl)-1,3-benzenediol

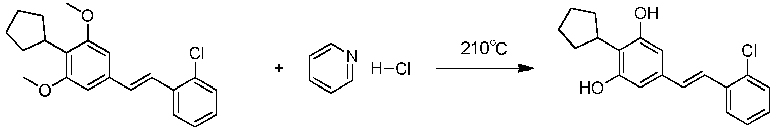

Referring to the synthesis of (*E*)-2-cyclopentyl-5-styryl-1,3-benzenediol in **Example 3,** a starting material (*E*)-2-cyclopentyl-5-(2-chlorostyryl)-1,3-dimethoxybenzene (450 mg, 1.31 mmol) was used for reaction, and the reaction solution was purified to give a product (*E*)-2-cyclopentyl-5-(2-chlorostyryl)-1,3-benzenediol (185 mg). Yield: 44.8%.

¹H NMR (400 MHz, DMSO-*d*₆) *δ* 9.21 (s, 2H), 7.95-7.92 (d, 1H),7.53-7.50 (m, 1H), 7.42-7.38 (d, *J* = 16.0 Hz, 1H), 7.35-7.31 (m, *J* = 16.0 Hz, 1H), 7.29-7.25 (d, *J* = 16.0 Hz, 1H), 7.15-7.11 (d, *J =* 16.0 Hz, 1H), 6.56 (s, 2H), 3.57-3.47 (m, 1H), 1.99-1.96 (m, 2H), 1.86-1.79 (m, 2H), 1.71-1.59 (m, 4H). ESI-MS *m*/*z* 313.1 [M-H]⁻.

### Example 29

### Synthesis of (E)-2-cyclopentyl-5-(3-chlorostyryl)-1,3-benzenediol

### (1) Synthesis of (E)-2-cyclopentyl-5-(3-chlorostyryl)-1,3-dimethoxybenzene

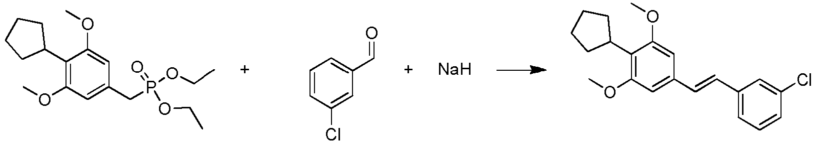

Referring to the synthesis of (*E*)-2-cyclopentyl-1,3-dimethoxy-5-styrylbenzene in **Example 3,** starting materials diethyl 4-cyclopentyl-3,5-dimethoxybenzylphosphonate (1.0 g, 2.81 mmol) and 3-chlorobenzaldehyde (522.4 mg, 4.21 mmol) were used for reaction, and the reaction solution was purified to give a product (*E*)-2-cyclopentyl-5-(3-chlorostyryl)-1,3-dimethoxybenzene (460 mg). Yield: 47.8%.

### (2) Synthesis of (E)-2-cyclopentyl-5-(3-chlorostyryl)-1,3-benzenediol

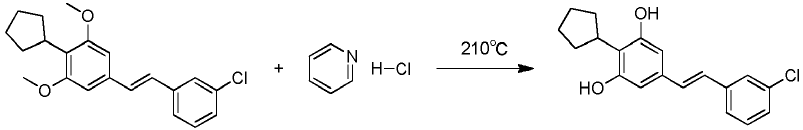

Referring to the synthesis of (*E*)-2-cyclopentyl-5-styryl-1,3-benzenediol in **Example 3,** a starting material (*E*)-2-cyclopentyl-5-(3-chlorostyryl)-1,3-dimethoxybenzene (460 mg, 1.34 mmol) was used for reaction, and the reaction solution was purified to give a product (*E*)-2-cyclopentyl-5-(3-chlorostyryl)-1,3-benzenediol (170 mg). Yield: 40.2%.

¹H NMR (400 MHz, DMSO-*d*₆) *δ* 9.19 (s, 2H), 7.74-7.73 (t, 1H), 7.59-7.57 (d, *J =* 16.0 Hz, 1H), 7.44-7.40 (t, *J =* 16.0 Hz, 1H), 7.35-7.32 (t, *J =* 16.0 Hz, 1H), 7.19-7.15 (d, *J =* 16.0 Hz, 1H), 6.94-6.90 (d, *J =* 16.0 Hz, 1H), 6.54 (s, *J* = 16.0 Hz, 2H), 3.54-3.47 (m, 1H), 2.05-1.99 (m, 2H), 1.86-1.78 (m, 2H), 1.69-1.59(m, 4H). ESI-MS *m*/*z* 313.1 [M-H]⁻.

### Test Example 1

The compounds of the present disclosure were tested for their activity, with the approved aryl hydrocarbon receptor agonist agent benvitimod and the commonly used aryl hydrocarbon receptor agonist compound FICZ selected as reference compounds.

### 1. Cytotoxicity test for test compounds

### 1) Cytotoxicity test for test compounds in PBMCs

The test compounds are shown in Table 1:

### (1) Formulation

Preparation of drug liquids containing culture medium at highest concentration: 1 µL of compounds 1# to 8#, reference 1#, reference 2#, and reference 3# at an initial concentration of 1 mM were separately added to 499 µL of culture medium to give a drug-containing culture medium at a concentration of 2 µM.

### (2) Preparation of cells

A. Cells: PBMC 5Million ID#: LP181017 CAT#: PB003F-5M
B. Thawing: PBMCs were taken out from the liquid nitrogen tank and quickly transferred to warm water at 37 °C for thawing. The cells were then transferred into a centrifuge tube containing a culture medium and centrifuged at 1200 rpm for 11 min. The centrifuge tube was taken out, and the culture medium was discarded. 5 mL of the culture medium was added with a pipette to resuspend the cells;
C. Cell counting: The cell counting software was turned on, and the cell type PBMC was selected. The viable cell concentration was 3.47 × 10⁵ cells/mL.

### (3) Cell treatment

Cell plating and treatment: The cell suspension was diluted to 7 mL, and the cells were plated in a 96-well plate. The cell suspension was added at 100 µL/well, and then the 2 µM drug liquids of compounds 1# to 8#, control 1#, control 2#, and control 3# were separately added at 100 µL/well in triplicate. The final viable cell count was about 2.5 × 10⁴ per well, and the drug concentration was 1 µM.

### (4) Cytotoxicity test

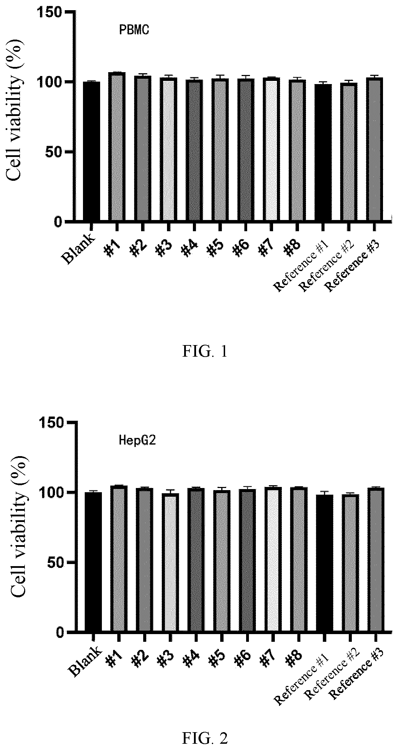

After 24 h of treatment, CCK8 was added at 10 µL/well, and after 3 h of reaction, the cells were detected on a microplate reader for the absorbance at wavelength 450 nm. The reading results are shown in FIG. 1.

The cell viability after treatment for the compounds was normalized by the absorbance of the control group.

### 2) Cytotoxicity test for test compounds in HepG2

### (1) Formulation

Preparation of drug liquids containing culture medium at highest concentration: 1 µL of compounds 1# to 8#, reference 1#, reference 2#, and reference 3# at an initial concentration of 1 mM were separately added to 499 µL of culture medium to give a drug-containing culture medium at a concentration of 2 µM.

### (2) Preparation of cells

Source: HepG2 cells were from the China National Collection of Authenticated Cell Cultures.

Thawing: HepG2 cells were taken out from the liquid nitrogen tank and quickly transferred to warm water at 37 °C for thawing. The cells were then transferred into a centrifuge tube containing a culture medium and centrifuged at 1200 rpm for 11 min. The centrifuge tube was taken out, and the culture medium was discarded. 5 mL of the culture medium was added with a pipette to resuspend the cells.

### (3) Cell treatment

The cell suspension was diluted to 7 mL, and the cells were plated in a 96-well plate. The cell suspension was added at 100 µL/well, and then the 2 µM drug liquids of compounds 1# to 8#, control 1#, control 2#, and control 3# were separately added at 100 µL/well in triplicate. The final viable cell count was about 2.0 × 10⁴ per well, and the drug concentration was 1 µM.

### (4) Cytotoxicity test

After 24 h of treatment, CCK8 was added at 10 µL/well, and after 3 h of reaction, the cells were detected on a microplate reader for the absorbance at wavelength 450 nm. The reading results are shown in FIG. 2.

As can be seen in FIGs. 1 and 2, at the test concentration, all compounds exhibited no significant effect on the cell viability of both PBMCs and HepG2 cells.

### 2. AhR agonism assay for compounds

The AhR agonistic effect of the compounds was evaluated on the basis of the results from the reporter gene assay for the compounds.
(1) Cell transfection: HepG2 cells were seeded into a 6-cm cell culture dish for adherent culture overnight.
When the cell confluence reached 80%, the culture medium was replaced with a fresh one, and the transfection working solution was added for transfection overnight. The formula of the working solution is as follows: 1 mL of Opti-MEM + 7.5 µg of pCMV-AHR + 7.5 µg of pTK-3 X DRE-Luc + 30 µL of lipo6000.
(2) Cell plating: The transfected cells were digested, prepared into a cell suspension at a concentration of 3 × 10⁵ cells/mL, and then plated on a 96-well plate at 100 µL/well for adherent culture overnight.
(3) Formulation
Preparation of drug liquids containing culture medium at highest concentration: 1 µL of compounds 1# to 16#, reference 1#, reference 2#, and reference 3# at an initial concentration of 1 mM were separately added to 499 µL of culture medium to give a drug-containing culture medium at a concentration of 2 µM; 40 µL of the 2-µM drug-containing medium was taken and diluted to give 400 µL of 0.2 µM drug-containing culture medium.
(4) Cell treatment
The prepared drug-containing culture media and a blank culture medium were added to the 96-well plate described above at 100 µL/well. After 16 h of treatment, a luciferase reporter gene assay was performed.
(5) The results of the assay were summarized, as shown in Table 1

**Table 1. Results of AhR activity assay for test compounds**

| Compound No. | Corresponding example | Compound structure | AhR receptor agonistic fold | |
|---|---|---|---|---|
| | | | Drug concentration 0.1 µM | Drug concentration 1 µM |
| 1# | Example 3 | 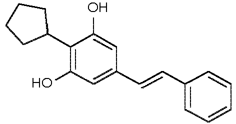 | 3.1±0.3 | 12.1±0.4 |
| 2# | Example 4 | 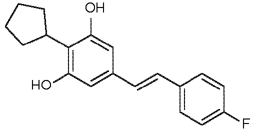 | 6.1±0.6 | 37.9±6.9 |
| 3# | Example 5 | 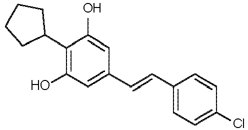 | 4.0±0.5 | 18.2±0.6 |
| 4# | Example 6 | 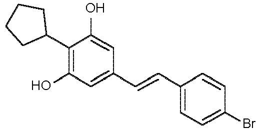 | 7.8±0.1 | 14.4±0.3 |
| 5# | Example 8 | 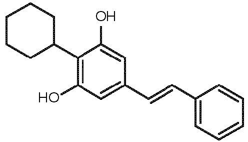 | 5.8±0.3 | 16.6±0.7 |
| 6# | Example 9 | 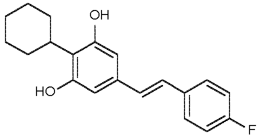 | 6.6±0.1 | 25.9±0.4 |
| 7# | Example 10 | 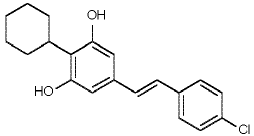 | 5.1±0.4 | 9.5±0.5 |
| 8# | Example 11 | 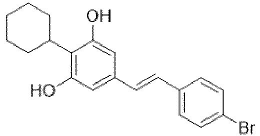 | 5.2±0.2 | 11.8±0.7 |
| 9# | Example 14 | 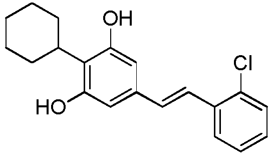 | 4.9±1.9 | 17.1±2.5 |
| 10# | Example 15 | 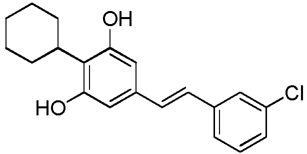 | 4.5±1.0 | 14.7±2.3 |
| 11# | Example 19 | 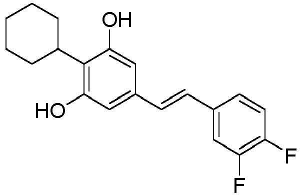 | 6.6±1.6 | 20.0±1.6 |
| 12# | Example 20 | 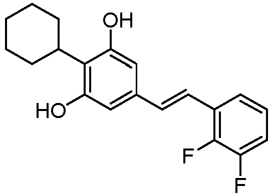 | 6.8:!:0.1 | 17.1±0.6 |
| 13# | Example 21 | 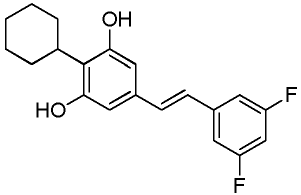 | 9.2±1.2 | 14.6±0.9 |
| 14# | Example 23 | 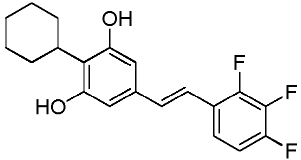 | 13.1±2.4 | 20.8±1.4 |
| 15# | Example 24 | 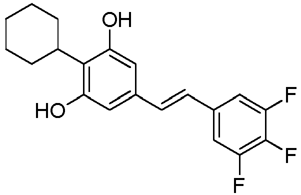 | 7.5±1.8 | 22.2±2.8 |
| 16# | Example 26 | 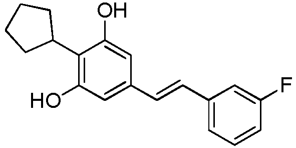 | 6.0±0.8 | 18.5±2.9 |
| Reference 1# | --- | Benvitimod | 3.2±1.2 | 10.6±1.0 |
| Reference 2# | --- | 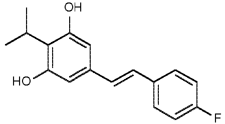 | 2.5±1.1 | 7.8±1.0 |
| Reference 3# | --- | AhR activator FICZ | 2.3±0.2 | 4.3±0.4 |

| | | | | |
|---|---|---|---|---|
| Note: The AhR receptor agonistic folds of the groups above are the fluorescence intensity ratios of the treatment groups to the blank. | | | | |

As can be seen from the data in Table 1, compounds 1-16# of the present disclosure all exhibited AhR activity, while test compounds 2# to 16# exhibited good AhR agonistic effects.

### 3. Regulatory effects on cytokine secretion in PBMCs by compounds

(1) Cells: Bone marrow-derived mononuclear cells;
(2) Cell plating and treatment: After the PBMCs were taken out from the liquid nitrogen tank and thawed, 80 µL of culture medium was added to a 96-well plate, and the cells were added at 200,000 cells/well. The culture media containing 2 µM or 0.2 µM compound 2#, compound 6#, or reference 1# were added to the 96-well plate at 80 µL, at a final drug concentration of 1 µM or 0.1 µM per well. The plate was let stand for 1 h for pretreatment. After the pretreatment, the drug-containing medium was discarded, and 1 µM or 0.1 µM solution containing 15 µg/mL PHA (phytohemaggluyinin) was added at 80 µL/well. After 24 h of stimulation, the content of cytokines in the culture medium was detected.

Blank control group: The cells were treated with a normal culture medium with no drugs;
PHA group: A culture medium containing 15 µg/mL PHA but no compounds of the present disclosure was added at 80 µL/well.

**Table 2. Results**

| Indicator | TNFa (pg/mL) | | IL4(pg/mL) | |
|---|---|---|---|---|
| Blank control group | 447.79 | | Below limit of detection, about 0 | |
| PHA group | 3649.21 | | 9.42 | |

| Treatment group | 0.1µM | 1µM | 0.1µM | 1µM |
|---|---|---|---|---|
| 2# | 1353.72 | 941.41 | 5.19 | 4.30 |
| 6# | 1433.29 | 810.71 | 4.62 | 2.75 |
| Reference 1# | 2559.02 | 2426.93 | 9.30 | 5.63 |

The results show that compounds 2# and 6# inhibited the cytokines TNFa and IL4 at concentrations of 0.1 µM and 1 µM, and the inhibitory effects of compounds 2# and 6# on TNFa and IL4 are significantly greater than those of reference 1# (benvitimod), particularly 2 folds greater at 1 µM.

### 4. Effect on DSS-induced colitis by compounds

(1) Animals: 40 C57BL/6 mice, male, aged 8 weeks, 22-24 g
(2) Materials: Dextran sodium sulfate (DSS) (MP.Biomedicals, USA, Cat. No. S7102); reference 1#, compound 6#; dimethylsulfoxide (DMSO); Tween 80; Wahaha purified water
(3) Procedures: The 40 mice were randomized into 4 groups of 10 mice each, including the blank group, the modeling group, the reference 1# group, and the compound 6# group. The normal blank group was given free access to distilled water, while the other 3 groups were given free access to a 2.5% aqueous DSS solution for 8 consecutive days. The solution was inspected daily and replaced with fresh DSS solution when necessary. On day one after modeling, the treatment was given. The normal blank group was given a normal saline, the colitis modeling group was given the solvent system, and the reference 1# and compound 6# groups were given the corresponding compounds at a dose of 10 mg/kg by intragastric administration. The body weight was recorded daily, the mice were observed for diarrhea and hematochezia, and the scores were recorded. On day 9, the mice were sacrificed, and the blood and colon were collected. The colon length, occult blood in the cecal contents, and other conditions were also recorded.

Test compound formulation: 1.00 mg of compound powder was dissolved in 20 µL of DMSO completely. Then, 20 µL of Tween 80 was added, and the mixture was well mixed. 0.96 mL of Wahaha purified water was added, and the mixture was well mixed to give a 1 mg/mL solution, which was administered at a dose of 0.1 mL/10 g (body weight).

Solvent: 20 µL of DMSO, 20 µL of Tween 80, and 0.96 mL of Wahaha purified water were mixed well (used in the modeling group).

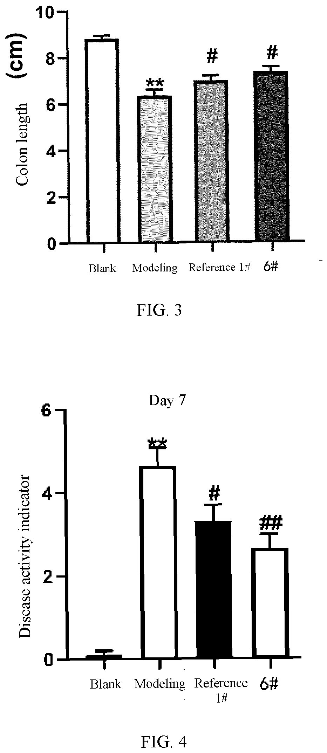

(4) Results: As shown in FIG. 3, the colon length summary shows that in the compound 6# and reference 1# benvitimod groups, the conditions were improved, while the effect in the compound 6# group was superior to that in the reference 1# benvitimod group.

As shown in FIG. 4, the disease activity indicator (a comprehensive score based on body weight change and hematochezia) data show that the compound 6# and the reference 1# benvitimod groups both exhibited good therapeutic effects, while the compound 6# group exhibited a more significant effect.

### 5. Effect on atopic dermatitis

### (1) Atopic dermatitis modeling

56 clean-grade BALB/C mice aged 6-8 weeks were subjected to 5 days of adaptive feeding and then randomized into 7 groups of 8 mice each:
A. Blank control group
B. Modeling + solvent group
C. Modeling + blank matrix group
D. Modeling + positive drug mometasone furoate group
E. Modeling + 1 wt% of benvitimod group
F. Modeling + 1.2 wt% of compound 2# group (prepared with blank matrix)
G. Modeling + 1.2 wt% of compound 6# group (prepared with blank matrix)

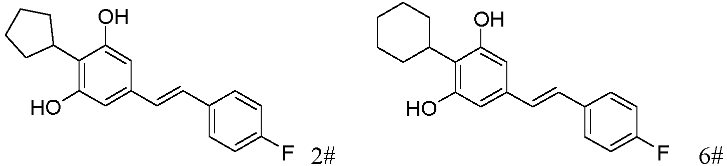

As shown in the following procedures, after depilation with rosin paraffin, 50 µL of 0.5% (W/V) 2,4-dinitrofluorobenzene (DNFB; solvent: a mixture of acetone and olive oil (acetone:olive oil = 3:1)) was smeared on the back of each mouse, and on days 4, 6, and 8, 50 µL of 0.25% (W/V) DNFB was administered in the morning, while an equal amount of solvent control was given to the blank control group. The animal experiments were conducted in accordance with the Experimental Animal Feeding, Management and Use Guidelines of China Pharmaceutical University and in compliance with animal study ethics and regulations.

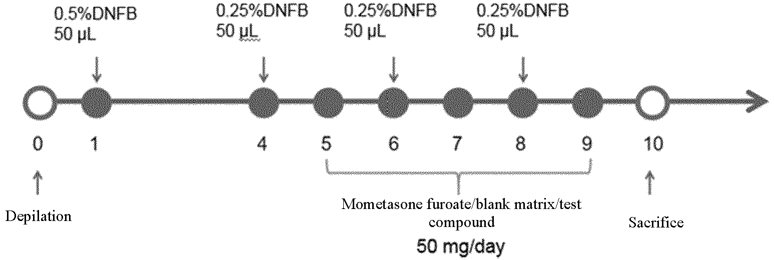

### Days

### (2) Regimen

As shown in the above procedures, the solvent control or DNFB was given in the morning for modeling; from day 5, the test compound ointment was administered once every afternoon at 50 mg/dose evenly on the skin surface. The specific administration method is as shown above; Specific procedures: As shown in the grouping described above, group B was given the solvent (acetone:olive oil = 3:1) at 50 mg once every afternoon from day 5; group C was given the blank matrix at 50 mg once every afternoon from day 5; groups D, E, F, and G were given the positive reference mometasone furoate, 1% benvitimod, 1.2% compound 2#, or 1.2% compound 6# at 50 mg, respectively, according to the grouping on day 5.

### Severity of atopic dermatitis was scored using the AD Score criteria

The severity of the appearance of dermatitis was evaluated based on 4 indicators: A. erythema/bleeding; B. dryness; C. effusion/crusting; D. edema. Each indicator was scored on 0-3, and the total score was acquired by adding the scores of the 4 indicators. The AD Score criteria are as follows: 0, no symptoms; 1, mild symptoms; 2, moderate symptoms; 3, severe symptoms. The skin on the back of each mouse was photographed and scored on days 0, 4, 6, 8, and 10 after modeling.

**Table 3. Back AD scores of mice in each group**

| Days | Blank control | DNFB+ | | | | | |
|---|---|---|---|---|---|---|---|
| | | Solvent group | Blank matrix | Mometasone furoate | Benvitimod | 2# | 6# |
| 0 | 0.00±0.00 | 0.00±0.00 | 0.00±0.00 | 0.00±0.00 | 0.00±0.00 | 0.00±0.00 | 0.00±0.00 |
| 4 | 0.00±0.00 | 2.94±0.63** | 3.95±0.90** | 3.50±0.61** | 3.63±0.78** | 3.19±0.75** | 3.50±0.35** |
| 6 | 0.00±0.00 | 6.88±2.45** | 7.31±1.82** | 5.38±1.65**^{b} | 7.44±1.07** | 5.63±1.96** | 5.56±1.45** |
| 8 | 0.00±0.00 | 8.75±1.82** | 8.13±0.99** | 6.50±1.22**^{a} | 7.19±1.14** | 6.13±1.96**^{aab} | 6.69±1.64**^{a} |
| 10 | 0.00±0.00 | 7.75±1.89** | 7.56±0.88** | 5.00±1.00**^{aabb} | 6.81±1.00** | 5.31±1.87**^{aab} | 5.44±1.13**^{aab} |

| | | | | | | | |
|---|---|---|---|---|---|---|---|
| Note: **: p < 0.01, vs blank control group; ^{a}: p < 0.05, vs solvent group (+ DNFB); ^{aa}: p < 0.01 vs. solvent group (+ DNFB); ^{b}: p < 0.05, vs blank matrix (+ DNFB); ^{bb}: p < 0.01, vs blank matrix (+ DNFB); the data were analyzed using two-way analysis of variance and Dunnett's double comparison test. | | | | | | | |

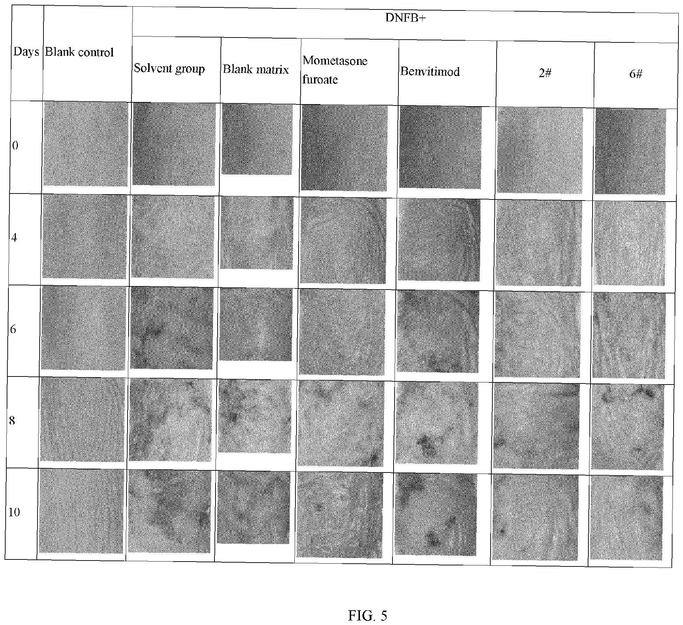

The skin inspection graphs are shown in FIG. 5.

### 6. Drug permeation rate test

### Procedures

**Test compounds:** compound 2# ointment, 1.2 wt%; compound 6# ointment, 1.2 wt%; benvitimod ointment, 1.0 wt%; 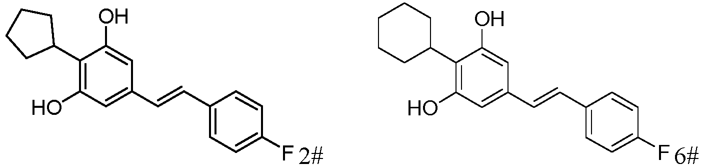
(1) Preparation of semipermeable membrane: The semipermeable membrane, which had been prepared in advance, was taken out from the refrigerator;
(2) Preparation of receiving liquid: 100 mL of polyethylene glycol 400 solution was brought to a final volume of 250 mL with normal saline. The mixture was mixed well and stored at 4 °C. Before each permeation in the receiving compartment, the system was preheated to 32 °C;
*(3) In vitro* diffusion test: A drug transdermal diffusion apparatus was used to fix the processed semipermeable membrane between the supply and receiving compartments. A constant temperature water bath circulation at 32 °C was maintained. The system was equilibrated for 30 min. Upon each replacement of the receiving liquid, the air at the lower layer was exhausted. About 0.5 g of the ointment was evenly smeared in the supply compartment. The system was stirred with a magnetic stirring at a rotation speed of 600 r/min. At 0.5, 1, 2, 4, 6, and 24 h, 1 mL of sample liquid was taken out from the receiving compartment, and simultaneously, an equal amount of fresh receiving liquid at the same temperature was added;

The sampling time points were 0.5, 1, 2, 4, 6, and 24 h, and 1 mL of sample was directly transferred into a liquid phase vial. Simultaneously, an equal amount of fresh receiving liquid at the same temperature was added. The liquid phase detection was conducted to calculate the drug permeation rate.

### Ointment-diffusion test-rabbit skin

### Procedures

(1) Preparation of *ex vivo* rabbit skin
(2) Preparation of receiving liquid: 100 mL of polyethylene glycol 400 solution was brought to a final volume of 250 mL with normal saline. The mixture was mixed well and stored at 4 °C. Before each permeation in the receiving compartment, the system was preheated to 32 °C.
*(3) In vitro* transdermal test: A drug transdermal diffusion apparatus was used to fix the processed rabbit skin between the supply and receiving compartments, with the stratum corneum facing the supply compartment. A constant temperature water bath circulation at 32 °C was maintained. The system was equilibrated for 30 min. Upon each replacement of the receiving liquid, the air at the lower layer was exhausted, and the liquid droplets on the skin surface were removed with paper. 0.5 g of the ointment was evenly smeared in the supply compartment. The system was stirred with a magnetic stirring at a rotation speed of 600 r/min. At 0.5, 1, 2, 3, 4, 5, 7, 9, 12, and 24 h, 1 mL of sample liquid was taken out from the receiving compartment and transferred into a liquid phase vial, and simultaneously, an equal amount of fresh receiving liquid at the same temperature was added. The liquid phase detection was conducted to calculate the drug permeation rate.

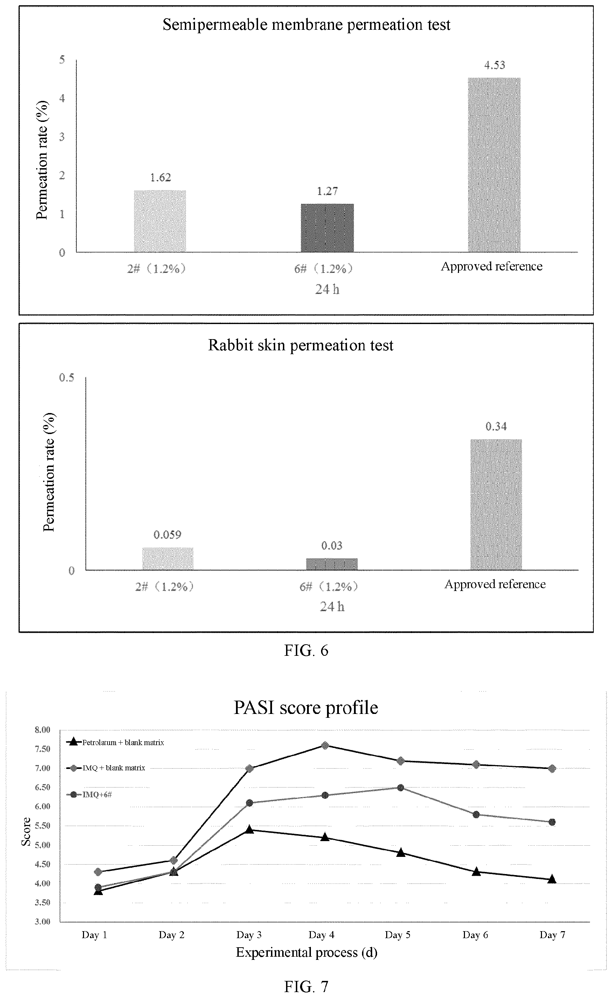

### The detection results for the 24-hour drug permeation rate are shown in FIG. 6.

As shown in FIG. 6, in the two test systems, the 24-hour drug permeation rate profiles of the three ointments were consistent, with the approved reference benvitimod > 2# > 6#. The results show that the ointments prepared from compounds 2# and 6# of the present disclosure exhibited lower transdermal absorptions, lower exposures in circulation, and milder lighter systemic adverse effects compared with the approved reference benvitimod.

### 7. Test for psoriasis in mice

1) The efficacy on IMQ-induced psoriasis in mice by compound 6# was evaluated. Compound structure: 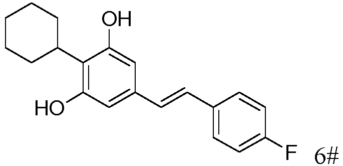
2) Grouping
   (1) Petrolatum + blank matrix group, 10 mice;
   (2) IMQ + blank matrix group, 10 mice;
   (3) IMQ + compound 6# group (1.2 wt% in blank matrix), 10 mice.
   IMQ: imiquimod
3) Formulation
   IMQ: 5% imiquimod (imiquimod was used for inducing psoriasis on the skin of modeling mice) 1.2% compound 6# formulation was prepared in blank matrix
4) Animals
   Species and strain: SPF-grade KM mice, male
   Weight: 35-40 g
   Source: SPF (Beijing) Biotechnology Co., Ltd.
   Housing conditions: air-conditioned room, with a temperature of 18-26 °C and a relative humidity of 20%-60%.
5) Procedures

Skin pretreatment: Mice were given free access to water and food. After 5 days of adaptation in the animal room, the hair on the back of the mice within an area of 2 cm × 2.5 cm was shaved. The area was treated with a depilatory cream for 30 s-1 min, and then the cream was removed with cotton pads. Randomization: 24 h after depilation, 75 mice were randomized into 4 groups by weight: petrolatum + blank matrix group (10 mice); IMQ + blank matrix group (10 mice); IMQ + compound 6# group (1.2% compound in blank matrix) (10 mice).

Modeling and treatment: 62.5 mg of 5% imiquimod or petrolatum in the morning was administered to the mice in the morning. In the afternoon, 62.5 mg of blank matrix or 1.2% compound 6# was smeared on the back.

Recording: Before modeling in the morning, the condition of the skin folds, erythema, and scales was observed, scored, and photographed daily, and the mouse PASI score was calculated.

**Table 4. Scoring criteria**

| Score | Severity |
|---|---|
| 0 | No symptoms (smooth skin without folds/scales/erythema) |
| 1 | Mild (mild folds on the skin at the edge of the treated area/mild scales on the skin at the treated area/mild redness on the skin at the treated area) |
| 2 | Moderate (mild folds/scales/redness on all treated skin areas) |

**Table 5. PASI score (a comprehensive score based on folds, scales, and erythema)**

| Group | Score | | | | | | |
|---|---|---|---|---|---|---|---|
| | Day 1 | Day 2 | Day 3 | Day 4 | Day 5 | Day 6 | Day 7 |
| Petrolatum + blank matrix | 3.80 | 4.30 | 5.40 | 5.20 | 4.80 | 4.30 | 4.10 |
| IMQ + blank matrix | 4.30 | 4.60 | 7.00 | 7.60 | 7.20 | 7.10 | 7.00 |
| IMQ + compound 6# | 3.90 | 4.30 | 6.10 | 6.30 | 6.50 | 5.80 | 5.60 |
| IMQ + compound 6# VS IMQ + blank matrix | 0.1069 | 0.3913 | 0.1296 | 0.0046 | 0.0314 | 0.0026 | 0.0002 |

The statistical difference between the IMQ + compound 6# group and the IMQ + blank matrix group was calculated by a T-test method; *p < 0.05, **P < 0.01, ***P < 0.001

The test results are shown in Table 5 and FIG. 7. The significant difference P value between the IMQ + compound 6# group and the IMQ + blank matrix group on day 7 was 0.0002, indicating a significant difference. That is, compound 6# of the present disclosure has a significantly superior therapeutic effect to that of the modeling control group IMQ + blank matrix.

## Claims

1. A compound of formula (I), or a pharmaceutically acceptable salt, a tautomer, or a stereoisomer thereof: 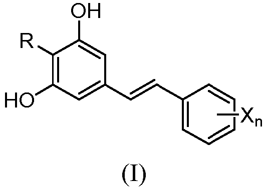
wherein X is selected from halogen, and n is 1-5;
R is selected from substituted or unsubstituted 3- to 7-membered cycloalkyl or cycloalkenyl, and substituted or unsubstituted 3- to 7-membered heterocyclyl;
the substitution is a substitution with at least one of C₁₋₆ alkyl, C₁₋₆ alkoxy, and halogen.

2. The compound, or the pharmaceutically acceptable salt, the tautomer, or the stereoisomer thereof according to claim 1, wherein X is F and/or Cl.

3. The compound, or the pharmaceutically acceptable salt, the tautomer, or the stereoisomer thereof according to claim 1, wherein the substituted or unsubstituted C₃-C₇ heterocyclyl is a N-containing heterocyclyl; the substitution is a substitution with at least one of C₁₋₆ alkyl, C₁₋₆ alkoxy, and halogen.

4. The compound, or the pharmaceutically acceptable salt, the tautomer, or the stereoisomer thereof according to any one of claims 1-3, wherein the compound, or the pharmaceutically acceptable salt, the tautomer, or the stereoisomer thereof is selected from the following compounds:

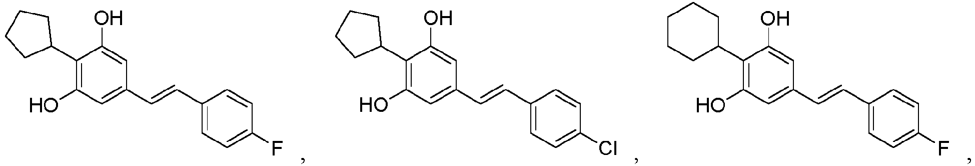

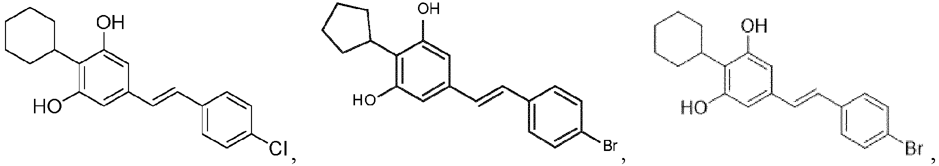

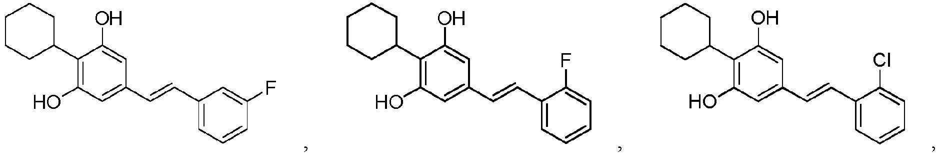

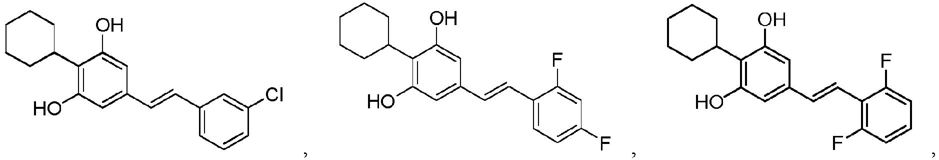

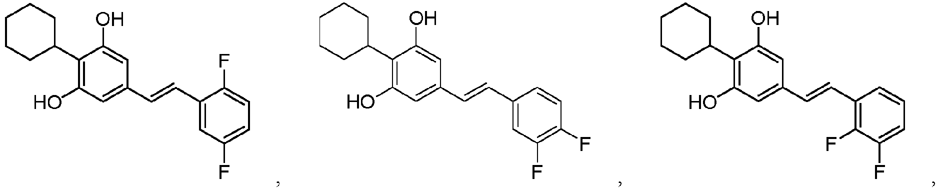

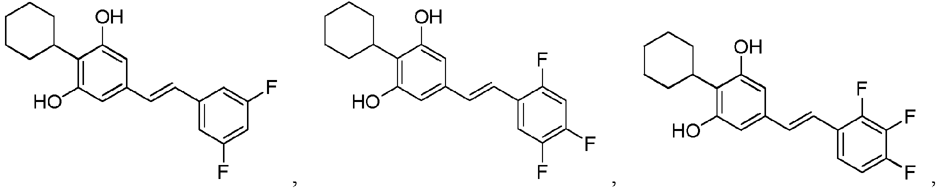

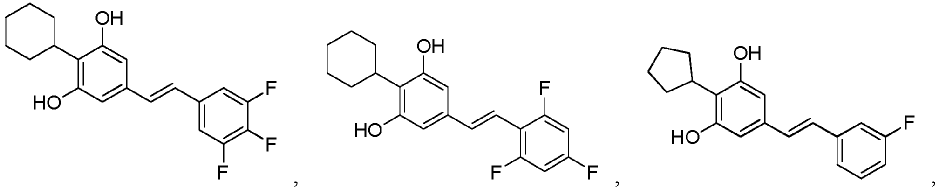

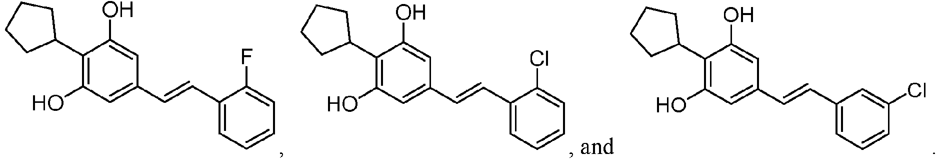

5. A compound of formula (II), or a pharmaceutically acceptable salt, a tautomer, or a stereoisomer thereof:

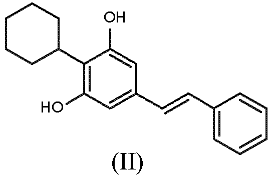

6. Use of at least one of the compound of formula (I), or the pharmaceutically acceptable salt, the tautomer, or the stereoisomer thereof according to any one of claims 1-4, and the compound of formula (II), or the pharmaceutically acceptable salt, the tautomer, or the stereoisomer thereof according to claim 5 for the manufacturing of a medicament for the treatment of a cancer, an autoimmune dysregulation, and other disorder with an immunological factor;
or for the prevention and/or treatment of a disease or condition mediated by the aryl hydrocarbon receptor (AhR);
or for the regulation of an immune- or inflammation-associated cytokine selected from IL-2, IL-3, IL-4, IL-5, IL-6, IL-10, IL-12, IL-13, IL-17, IL-22, IL-23, TNFα, TGF-β, IFN-γ, and IL-1β, and for the prevention and/or treatment of a disease caused by the abnormality of the cytokine described above.

7. The use according to claim 6, wherein the autoimmune dysregulation disorder is selected from one or more of psoriasis, eczema, atopic dermatitis, multiple sclerosis, systemic lupus erythematosus, inflammatory bowel disease, ulcerative colitis, rheumatoid arthritis, chronic kidney disease, ankylosing spondylitis, Sjogren's syndrome, polymyositis, vasculitis, polymyalgia rheumatica, immune thrombocytopenia, dry eye syndrome, type 1 diabetes, psoriasis, and arthritis.

8. The use according to claim 6, wherein the disorder with the immunological factor is selected from one or more of asthma, hypersensitivity, infection, osteoporosis, atherosclerosis, type 2 diabetes, graft-versus-host disease, and transplant rejection.

9. A pharmaceutical composition, comprising a pharmaceutically acceptable carrier or excipient and at least one of the compound, or the pharmaceutically acceptable salt, the tautomer, or the stereoisomer thereof according to any one of claims 1-4, and the compound of formula (II), or the pharmaceutically acceptable salt, the tautomer, or the stereoisomer thereof according to claim 5.

10. A method for preparing the compound according to any one of claims 1-4, comprising:
reacting compound IMe with compound SMc to give compound IMf, and reacting compound IMf in an acidic condition to give a compound represented by formula (I); 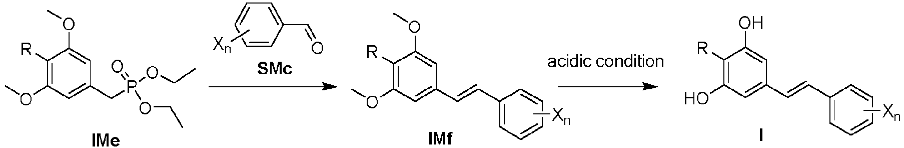
wherein X is selected from halogen, and n is an integer of 1-5;
R is selected from substituted or unsubstituted 3- to 7-membered cycloalkyl or cycloalkenyl, and substituted or unsubstituted 3- to 7-membered heterocyclyl; the substitution is a substitution with at least one of C₁₋₆ alkyl, C₁₋₆ alkoxy, and halogen.
